# EUROPEAN PATENT APPLICATION

(11) **EP 1 536 005 A1**
(43) Date of publication of application: **01.06.2005**
(21) Application number: 03788129.9
(22) Date of filing: 15.08.2003
(51) Int. Cl.: C12N 15/09, C12N 15/70, C12Q 1/02, G01N 33/53

(54) **METHOD OF ASSAYING INTERACTION BETWEEN PROTEINS**

(30) Priority: 16.08.2002 JP 2002237411
(71) Applicant: The University of Tokyo, Tokyo 113-8654 (JP)
(72) Inventor: UEDA, Hiroshi, Nagareyama-shi, Chiba 270-0111 (JP); NAGAMUNE, Teruyuki, Kawagoe-shi, Saitama 350-0808 (JP)
(74) Representative: Wibbelmann, Jobst, Dr., Dipl.-Chem.
(86) International application number: PCT/JP2003/010386
(87) International publication number: WO 2004/016782

(57) **Abstract**

A novel method for determining interaction between VH fragment and VL fragment of the variable region of an antibody is provided by the present invention. The method according to this invention can be widely used for detection of protein interactions. If a phagemid vector containing an amber codon is used for transformation of an amber suppressor strain of E.coli according to the method of the invention to produce phages, both of the VH fragment and the VL fragment will be displayed on the phage particles. In contrast, if the same vector is used to transformation of an amber non-suppressing strain of E.coli to produce phages, for the presence of the amber codon, only the VH fragment will be displayed on the phage particles, while the VL fragment will be secreted into the culture medium by the E.coli. Thus, display switch occurs. The interaction between the VH fragment and the VL fragment can be determined, by immobilizing the VL fragment secreted into the culture medium on a solid phase and quantifying binding between the VH fragment displayed by phages and the VL fragment.

## Description

### BACKGROUND OF THE INVENTION

### 1. Field of the Invention

The present invention relates to a method to determine the interaction between multiple proteins, particularly the interaction between VH fragment and VL fragment of the variable region of an antibody. Moreover, the present invention relates to a vector constructed for application in the invented method.

### 2. Description of the Related Art

The present inventors developed open sandwich ELISA method, a novel method for immunoassay that determines the concentration of an antigen indirectly by determining the interaction between VH/VL of an antibody, and disclosed the method in Japanese Patent Publication No.10-78436. Various immunoassays utilizing the specificity of antigen/antibody reaction allows detection of a minute amount of substance in a mixture at a high sensitivity. This open sandwich method is advantageous in comparison with the conventional sandwich ELISA in that the operation is easy, and it allows the determination of a monovalent antigen.

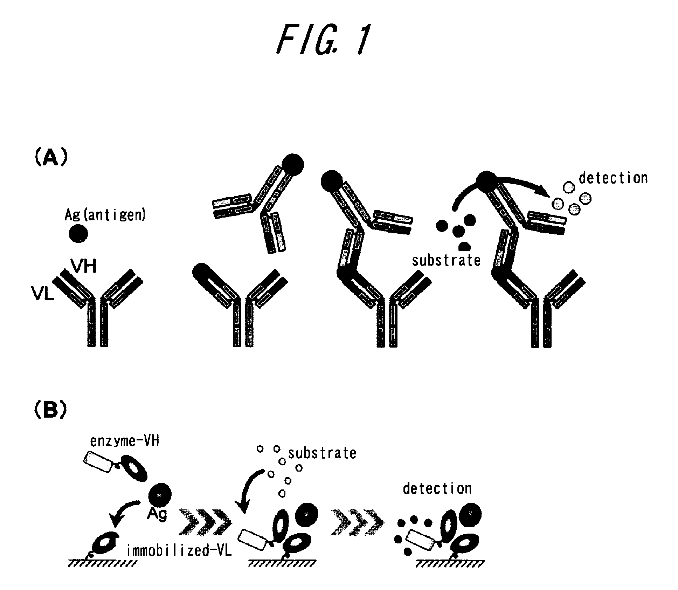

Fig. 1 schematically shows a comparison of the concept underlying the conventional sandwich ELISA method (A) and the concept underlying the open sandwich ELISA method (B) proposed by the present inventors. According to the sandwich ELISA method (A), a primary antibody specific to an antigen to be tested is immobilized on a solid phase. A solution containing the antigen and a secondary antibody labeled with an enzyme are added to the solid phase sequentially in this order. The system is washed, and the concentration of the antigen is determined by determining the enzymatic activity. On the other hand, according to the open sandwich ELISA method (B), the VL of an antibody specific to an antigen of interest is immobilized.
An antigen solution and the VH labeled with an enzyme are simultaneously added to the system and the concentration of the antigen is quantified by determining the enzymatic activity. In comparison with the sandwich ELISA method, the invented method can save one washing operation.

The antibody fragments VH, VL used for the open sandwich ELISA method should absolutely meet the following criteria: i) they should have high affinity toward the antigen of interest; and ii) interaction between VH and VL should be weak in the absence of the antigen. Thus, it is necessary to prepare antibody fragments that can satisfy the above-mentioned criteria. To establish the open sandwich ELISA method as an assay method or a detection system in the future, the method should become a system that can be applied for measurement of wide range of antigens. If a system is established and an antibody library with immense number of antibodies can be screened rapidly and easily for those satisfying above-mentioned criteria for every antigens, it would be highly beneficial for practical application of the open sandwich ELISA method.

To promote the practical application of the open sandwich ELISA method, it is highly desirable to utilize a system that enables rapid determination of the binding ability of an antibody to an antigen and VH/VL interaction in the presence or absence of the antigen, which is achieved by present invention. The present invention also has the purpose to provide a method that enables selection of antibodies that have higher response to an antigen from an antibody library.

A number of phage display techniques have been proposed heretofore. The phage display technique consists of displaying VH fragment and VL fragment of the variable region of various antibodies simultaneously on particles of a filamentous phage, evaluating the binding ability of the antibody to an antigen by its binding ability to the antigen, and selecting antibodies with higher binding activities. In many cases using such the phage display technique, a library is expressed or displayed as a fusion protein on the surface of pIII or pVIII, which are coat proteins of filamentous phage such as M13, fd, etc.

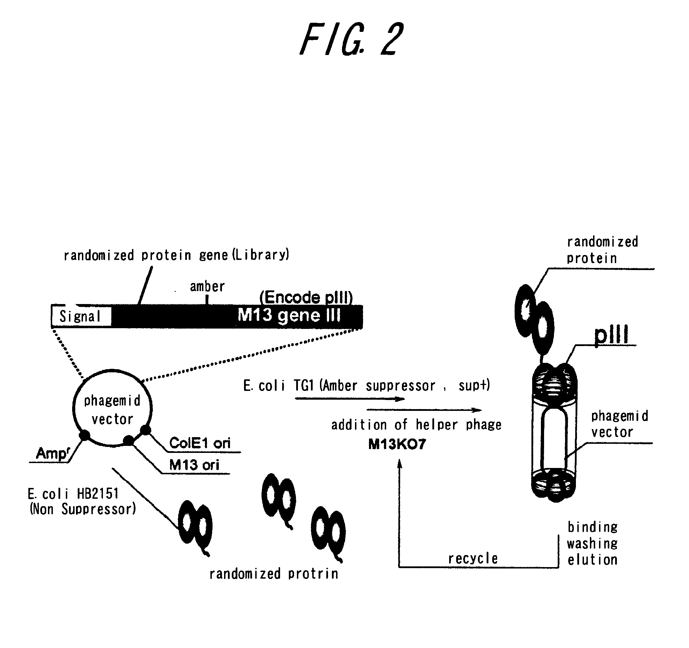

Fig. 2 shows the outline of a phage display technique using pIII. A phagemid vector is constructed in which a random sequence is introduced upstream of gene III coding for the coat protein pIII of the phage. Then E.coli is transformed by the vector and rescued by a helper phage, thereby a phage displaying proteins containing random sequences in the pIII can be obtained. The target molecule can be screened from the phage library thus produced and phages that specifically bind to the target molecule can be selected. The library of phages can be applied to a system where the target molecule has been immobilized, phages having high binding activities can be selected by performing procedures of binding, washing and elution (i.e. panning). The thus recovered phages are allowed to infect E.coli and proliferate, and the product is applied for the next cycle. By repeating this screening procedure, the ratio of phages with binding ability in the library can be increased effectively.

According to this selection system, following merits are ensured: sequence of the selected protein can be easy determined because its phenotype is linked with its genotype; the target protein can be concentrated efficiently because the amplification process is introduced during screening; and the proteins can be separated by changing the host strain to be infected. Suitable proteins to be displayed include various proteins including short random peptides composed of several amino acid residues, antibody fragments, proteases, human growth hormone, nucleic acid binding proteins, etc.

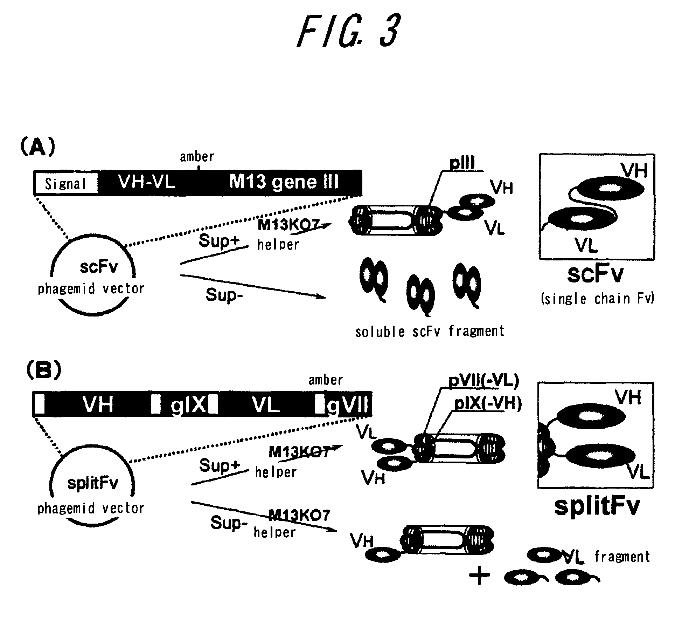

Generally, if it is required to display an antibody fragment in the phage display, the antibody is used in the form of scFv (single chain Fv) consisting of VH and VL connected by a linker. However, a new phage technique (Kim D. Janda et al., WO 00/71694 A1) was reported recently, wherein the VH fragment and the fragment are displayed separately by allowing the VH to be displayed on gVII protein and VL to be displayed on gIX protein. Fig. 3 schematically shows the outlines of the conventional method of displaying an antibody in the form of single chain antibody fragment (A), and above-mentioned method of displaying the VH fragment and the fragment separately (B).

The antibody library used for phage display mainly includes a naive library produced from V genes of an non-immunized animal, a synthetic library with artificial mutations introduced into hyper-variable regions of a naive library, and an immunized library prepared from an animal immunized with a target antigen.

The naive library is advantageous in that, it enables selection of antibodies for various antigens in principle, since V genes of IgM antibodies prior to occurrence of affinity maturation due to antigenic stimulation are utilized; and it enables construction of libraries of humanized antibodies since an immunization operation is not required. On the other hand, the naive library is disadvantageous in that, it is indispensable to increase the size of library in order to improve the affinity of antibodies, which requires much labor, and the library contains many unnecessary elements. The synthetic library comprises introduction of mutations in CDR of a naive library by the means of site-directed mutagenesis or PCR.

The immunization library is obtained by collecting antibody genes from a mouse immunized with a target antigen in advance, and thus its merit lies in that libraries with very high antigen specificity and binding activity can be obtained. However, its demerit lies in that immunization takes a long period (1-3 months), preparation of antibodies toward a toxic molecule or a self-molecule (a molecule incapable of induction of immune response) is difficult, and preparation of a humanized antibody is difficult.

As mentioned earlier, several techniques for selecting antibodies with binding activity (phage display technique) have been proposed so far, by displaying VH fragments and VL fragments of the variable region of various antibodies on the particles of the filamentous phage simultaneously, and evaluating the binding ability of the antibodies to the antigens by the binding abilities between antigens and the filamentous phage on which antibody fragments are displayed. According to these techniques, the binding activity of the overall variable region of an antibody including both of the VH and VL regions can be evaluated, however, evaluation of interaction between VH/VL can-not be achieved. Moreover, to obtain antibody fragments suited to be applied for the open sandwich ELISA described above, it is necessary to select a pair of VH and VL that exhibit weak interaction in the absence of the antigen.

### SUMMARY OF THE INVENTION

As a first means for solving the above-mentioned problems, the present invention provides a vector, the vector containing at least following five DNA sequences:
(1) a DNA sequence encoding one protein or its fragment; (2) a DNA sequence encoding a protein for displaying said one protein or its fragment on a phage; (3) a DNA sequence encoding another protein or its fragment; (4) a stop codon that enables display switch by a host strain; and (5) a DNA sequence encoding a protein for displaying said another protein or its fragment on the phage, the vector having a structure comprising these 5 DNA sequences in the order of (1), (2), (3), (4) and (5) or (3), (4), (5), (1) and (2) in the 5'-3' direction of the vector, by the presence of the stop codon that enables display switch by said host strain, when said vector is introduced into a suppressor-mutant host strain, the vector provides a two-protein displaying phage on which both of said one protein or its fragment and said another protein or its fragment are displayed, and when said vector is introduced into a non-suppressing host strain, the vector provides a one-protein displaying phage on which only said one protein or its fragment is displayed and said another protein or its fragment is secreted into the culture medium.

As a second means for solving the above problems, the present invention provides a method for determining interaction between one protein or its fragment and another protein or its fragment, the method comprising the steps of:
(1) transforming a non-suppressing host strain by using said vector, thereby obtaining one-protein displaying phage on which only said one protein or its fragment is displayed, and a culture medium containing said another protein or its fragment being secreted from said non-suppressing host strain;
(2) immobilizing said another protein or its fragment in the supernatant on an appropriate support;
(3) reacting said another protein or its fragment immobilized on the support with said one protein or its fragment displayed on the one-protein displaying phage, thereby said one protein or its fragment is bound to said another protein or its fragment; and
(4) determining the amount of immobilized phages by an immunoassay using a labeled anti-phage antibody, thereby evaluating the binding ability between said one protein or its fragment and said another protein or its fragment.

As a third means for solving the above problems, the present invention provides a method for obtaining a variable region of an antibody in which interaction between a VH fragment and a VL fragment alters by the presence of an antigen, the method comprising the steps of:
(1) transforming a suppressor-mutant host strain by using said vector containing DNA sequences encoding VH and VL fragments of the variable region of an antibody to obtain a VH/VL displaying phage, on which both the VH and VL fragments are displayed when the vector is introduced into the suppressor-mutant host strain;
(2) confirming the binding ability between the VH/VL displaying phage obtained in the step (1) and an antigen;
(3) transforming a non-suppressing host strain by using said vector whose ability to provide a VH/VL displaying phage is confirmed in the step (2), or transfecting a non-suppressing host strain with the VH/VL displaying phage obtained in the step (1), thereby obtaining a VH displaying phage on which only the VH fragment is displayed and a culture medium containing the VL fragment secreted by said non-suppressing host strain; or a VL displaying phage on which only the VL fragment is displayed and a culture medium containing the VH fragment secreted by said non-suppressing host strain;
(4) immobilizing said VL fragment or said VH fragment in the supernatant on an appropriate support;
(5) reacting said VL fragment immobilized on the support with said VH fragment displayed on the phage, or reacting said VH fragment immobilized on the support with said VL fragment displayed on the phage, in the presence or absence of an antigen;
(6) determining the amount of immobilized phages by an immunoassay using a labeled anti-phage antibody, thereby evaluating the binding ability between said VH fragment and said VL fragment; and
(7) determining that an antibody variable region in which interaction between the VH fragment and the VL fragment significantly alters in the presence of an antigen is obtained, provided that the binding ability between said VH fragment and said VL fragment in the presence of an antigen is two times or more than the binding ability between the VH fragment and the VL fragment in the absence of the antigen.

The present invention will be further detailed below by means of following descriptions and figures, but they should not be considered in any way to restrict the scope of the present invention.

### BRIEF DESCRIPTION OF THE DRAWINGS

Fig. 1 is a schematic diagram illustrating the principles of the sandwich ELISA and the open sandwich ELISA.
Fig. 2 is a schematic diagram illustrating the principle of the phage display method.
Fig. 3 is a schematic diagram illustrating the principles of the method for displaying a fragment of an antibody in the form of a single chain and the method for displaying the VH fragment and the VL fragment separately.
Fig. 4 is a schematic diagram illustrating the split Fv-coding region of the phagemid vector pKS.
Fig. 5 is a graph illustrating the result of investigation on antibody display by ELISA.
Fig. 6 is a graph illustrating the result of investigation on the antigen binding activity by ELISA.
Fig. 7 is a graph illustrating the result of the investigation on antigen (HEL) binding activity by ELISA with respect to scFv type and split Fv type phages displaying HyHEL-10 at phage concentration of 10¹⁰ cfu/ml.
Fig. 8 is a graph illustrating the result of investigation on the antigen (HEL) binding activity by ELISA with respect to scFv type and split Fv type phages displaying HyHEL-10 at phage concentration of 10⁹ cfu/ml.
Fig. 9 is graphs illustrating the results of open sandwich ELISA with respect to pKS1(HyHEL10)/sup+.
Fig. 10 is a schematic diagram illustrating the mechanism underlying the display switch in the experimental system according to invention.
Fig. 11 is a graph illustrating the result of open sandwich ELISA using HyHEL10 and D1.3.
Fig. 12 is a graph illustrating the ratio of positive clones accompanied with panning operation.
Fig. 13 is a schematic diagram illustrating the process for preparation of HyHEL-10 or D1.3 type split Fv fragments.
Fig. 14 is a schematic diagram illustrating the process from preparation of phage library to selection of clones with high HEL binding ability.
Fig. 15 is a figure showing the relationship between HEL binding ability and suitability for open sandwich ELISA.
Fig. 16 is a figure showing relationship between HEL binding activity and V_{H}/V_{L} interaction

### DETAILED DESCRIPTION OF THE PREFERRED EMBODIMENTS

The present invention provides a novel method that enables measurement of interaction between VH and VL of an antibody. The invented method allows the selection of antibodies suited for the purpose of application in the open sandwich ELISA. The invention comprises phagemid vectors for incorporation of cDNA fragments coding for an antibodies, suppressor E. coli cells or non-suppressing E.coli cells transformed by the vectors, phages produced by infecting helper phages to the E.coli cells, and the culture medium containing soluble antibody fragments.

After phagemid vectors incorporated with cDNA encoding variable region of an antibody having binding ability to the target substance (antigen), as well as VH and VL fragments are produced, amber-suppressor E.coli cells are transformed by the phagemid vectors, and phages are produced by infection of the helper phages according to conventional method. The phages display both the VH fragment and the VL fragment on their particles, and the binding ability of the variable region (VH + VL) to the antigen can be confirmed. When the same phages are used to transform non-suppressing E.coli cells to prepare phages, owing to the display switch described below in detail, the VL fragment is secreted into the culture medium and only the VH fragment is displayed on the phage and obtained in the supernatant in the same manner.

The culture medium of the cell is centrifuged, and the supernatant is then poured onto a microplate coated with protein L having binding ability towards the VL fragment. Since the VL fragment is immobilized on the microplate through this procedure, the amount of the phages immobilized on the plate alters depending on the strength of the VH/VL fragment interaction. It is readily possible to determine the interaction between the VH/VL fragments by enzyme-linked immunoassay (ELISA) using a peroxidase-labeled anti-phage antibody. Thus, according to the invented method, it is possible to evaluate the VH/VL interaction of an antibody much easier than the conventional methods. Moreover, according to the invented method, it is also possible to select antibody fragments with high binding ability to an antigen.

If an antigen is allowed to coexist at the measurement system, clones exhibiting significant alteration on the VH/VL interaction by the presence of the antigen can be screened rapidly. With the antibody fragments with weak VH/VL interaction, the VL fragments immobilized onto the support hardly exhibit direct binding to the VH fragments displayed on phages, and thus few phages can bind on the support. However, for some antibodies, in the presence of the antigen both the VH fragment and the VL fragment bind to an antigen together to form stable complex and phages can attach onto the support via the antigen/VL. Therefore, the amount of phages attached on the support can be determined by using, for example, an anti-phage antibody.
Then antibody fragments exhibiting alteration in the amount of bound phages in the presence of the antigen can be selected. Such antibody fragments are likely to exhibit significant alteration in their VH/VL interaction by antigen binding, and thus they are particularly suitable for open sandwich ELISA. If interaction between the VH fragment and the VL fragment of the variable region of an antibody is increased twice or more by existence of an antigen, such antibody fragments can be used for the purpose of the present invention (Suzuki et al., Anal. Biochem., 286:238-246 (2000)).

In the practice of the invented method, a phagemid vector is preferably used. The phagemid vector is a plasmid encoding the replication origin of a filamentous phage, therefore, after transformation of an E.coli cell with a phagemid vector, it is further necessary to infect the cell with a helper phage. Through this procedure, coat proteins necessary for the production of phage particles are provided, and phages harboring phagemid DNA can be obtained. The helper phage used in this procedure may include, most preferably, an M13KO7 helper phage used in the Example described below, but it is not limited to the phage. As an easier procedure, a phage vector containing a necessary DNA sequence may be used. In the case of a phage vector, it is possible to obtain target phages directly by infecting E.coli cells with the phage vector, and use of a helper phage is not necessary.

The term "display switch" used herein refers to the switch phenomenon of the following; if the vector is introduced into a suppressor-mutant host strain, both of said "one protein" and said "another protein" are displayed on the phage, whereas, if the vector is introduced into a non-suppressing host strain, the one protein is displayed on the phage while the another protein is secreted in the culture medium. In this Specification, we refer a phage displaying both of the proteins "two-protein displaying phage," while we refer a phage displaying one protein "one-protein displaying phage." When the "one-protein displaying phage" is obtained, said "one protein" is displayed on the "one-protein displaying phage", and a gene encoding said "another protein" is expressed in the E.coli cell to be secreted outside of the cell bodies.

If said protein is a fragment of the variable region of an antibody, particularly if a phage displays both the VH fragment and the VL fragment, such phage is referred to "VH/VL displaying phage." Moreover, if a phage displays the VH fragment and the VL fragment is secreted from E.coli into the culture medium, such culture medium is referred to "VH displaying culture medium". Furthermore, if a phage displays the VL fragment and the VH fragment is secreted into the culture medium, such culture medium is referred to "VL displaying culture medium".

Display switch described above becomes possible by the existence of a stop codon. The stop codon to be used for display switch may preferably include amber codon as used in the Example below. The amber codon is a codon composed of TAG, which is one of the stop codons for termination of protein synthesis. However, the stop codon used in the present invention is not limited to the codon, and other stop codons such as opal codon (TGA) or ochre codon (TAA) may also be used for the same purpose.

If a vector containing the amber codon is introduced into an amber suppressor strain of E.coli, the two-protein displaying phages as described above can be obtained, because mis-translation of the stop codon (amber suppression) is expected to occur at a certain probability, and thus fusion protein is produced in the body of bacterial cell. On the other hand, if the same vector is introduced into non-suppressing host cells, amber suppression does not occur, and thus the amber codon can be properly recognized as a stop codon. Then, expression of fusion protein does not occur, and the protein encoded by the gene existing upstream of the amber codon can be secreted outside of the cells, thereby display switch occurs. Display switch can be also caused by introducing the opal codon into a suppressor-mutant host strain that brings opal suppression, or by introducing the ochre codon into a suppressor-mutant host strain that brings ochre suppression.

Suitable suppressor-mutant host stains to be used in the purpose of the invention may include bacterial strains having sup E or gln V, for example, E.coli TG1 strain, XL1-Blue strain, DH5α strain, JM109 strain, NM522 strain, etc. Suitable non-suppressing host strains may include strains such as E.coli JM105 strain, NV1184 strain, HB2151 strain, etc. However, the suppressor-mutant host strain and non-suppressing host strain to be used in the invention are not limited to the strains mentioned above as long as the host starins have F' plasmid, which is necessary for filamentous phage infection. However, the above-mentioned host strains may be substituted for other host strains having equivalent function.

The vector used according to the invented method comprises (1) a DNA sequence encoding one protein or its fragment; (2) a DNA sequence encoding a protein for displaying said one protein or its fragment on a phage; (3) a DNA sequence encoding another protein or its fragment; (4) a stop codon that enables display switch by a host strain; and (5) a DNA sequence encoding a protein for displaying said another protein or its fragment on the phage. The vector may comprise above-mentioned (1), (2), (3), (4) and (5) in the order, or (3), (4), (5), (1) and (2) in the order.

The vector having such structure can display said "one protein" on the phage. On the other hand, when the vector is introduced into non-suppressing host strain, said "another protein" is not expressed on the phage as a fusion protein, but secreted out to the culture medium, because a stop codon capable of display switch exists between the "DNA sequence encoding another protein or its fragment" and the "DNA sequence encoding a protein for displaying said another protein or its fragment on the phage".

The suitable DNA sequence that can be used as the "DNA sequence encoding a protein for displaying a protein or its fragment on the phage" described above preferably includes DNA sequences encoding a surface protein of a filamentous phage. Such DNA sequences encoding a surface protein of a filamentous phage include DNA sequences encoding pVII protein, pIX protein, pIII protein, and pVIII protein. Particularly preferred is a DNA sequence encoding pVII protein or pIX protein of a filamentous phage as used in the Example below. However, the suitable DNA sequence is not limited to those described above, but other DNA sequences may be chosen and used as appropriate, as far as the DNA sequence encodes a protein capable of producing a fusion protein together with the target protein and available for the purpose of this invention.

The method of the invention is particularly suitable for determination of interaction between the VH fragment and the VL fragment of an antibody. However, theoretically, the invented method can be also applied for any multimeric proteins existing as a heterodimer, not only variable region fragments of an antibody, for the purpose to evaluate the interaction between the monomers comprising the heterodimer, by displaying said multimeric protein on the filamentous phage. If interaction between the "one protein" (which is one specific monomer) and the "another protein" (which is the another monomer) can be measured, it is valuable not only in the field of basic research, but also in the field of clinical diagnostics. Said "one protein" and said "another protein" mentioned in this invention should not be interpreted as limiting the scope of the invention, and the invented method provides a novel technique that enables measurement of interaction between proteins.

An antibody, exhibiting weak interaction in the absence of an antigen and strong interaction in the presence of an antigen, can be obtained by the invented method. Moreover, an assay kit, for example as described in the following, may be prepared by using such antibody.
1) Immobilize a VL fragment on a tube or a microplate by means of biotin/avidin interaction or physical adsorption.
2) Prepare a fusion protein comprising the VH fragment and a reporter enzyme (e.g., alkaline phosphatase) and allow it to contact with the support immobilized with the VL fragment together with a sample for a specified period.
3) Measure the activity of the immobilized enzyme after washing.
   The result is utilized as an index of the concentration of an antigen in the sample.

Another assay kit as follows can be also prepared.
1) Label the VH fragment and the VL fragment with two kinds of fluorescent dyes exhibiting overlapped absorption or fluorescence spectrum with each other (e.g., fluorescein and rhodamine).
2) Mix them with a sample, and excite only the short-wave fluorescent dye by its excitation light after approximately 5 minute. It is possible to detect the transition of fluorescence energy as a result of the association of VL and VH by measuring fluorescence intensities of the two kinds of fluorescent dyes. The ratio of the two fluorescence intensities may be used as an index of the antigen concentration in the sample. The latter method will enable the determination of the concentration of an antigen without requiring the washing step.

Furthermore, another assay kit as follows can be also prepared.
1) Make the VH fragment or the VL fragment be expressed in E.coli as a fusion protein with two kinds of enzyme fragments (e.g., LacZΔα and LacZΔω) and purify the product. The enzyme fragments may be those exhibiting no or weak activity in their single forms, while exhibiting enhanced activities when they become closer to each other.
2) Combine the two kinds of fusion proteins with a sample, leave the mixture for a specified period, then combine the mixture with a substrate (e.g., luminescent substrate such as Galacton Plus). Then measure the activity of the fusion protein complex, and use the measured value as an index of the concentration of an antigen in the sample. According to this method, the concentration of an antigen can be determined with sensitivity much higher than the two methods described above and washing procedure is not required in this method. (Yokozeki et al., Anal. Chem., 74(11):2500-2504, 2002).

The substances to be determined by the invented method may include, above all, specific proteins, peptides, various hormones, narcotics, and medicines in the serum examined in the clinical examination. Moreover, chemical substance and agricultural chemical in environmental water suspected of their toxicity, such as dioxin, bisphenol A and nonylphenol, may be also determined by this invented method.

In the Example described below, an investigation was conducted on whether display switch of VL was successfully achieved by using a proposed non-suppressing E.coli. More specifically, using anti-HEL (hen egg-white lysozyme) antibody named HyHEL-10, which had been shown to be effectively applied for the open sandwich ELISA by the present inventors, a model system for screening was constructed, and it was experimentally demonstrated that the proposed model could function properly. The phagemid vector pKS1 was constructed to display VH at the gIX protein and VL at the gVII protein separately (split Fv), and display switch of the VL caused by changing the host E.coli was confirmed by the open sandwich ELISA.

In the first selection step of the Example below, a VH/VL displaying phage was adopted. If an amber-suppressor strain such as TG1 or XL1-Blue was transformed with the produced vector, error in reading occurs on the amber codon at a certain probability and the corresponding position was replaced with glutamine. Fusion protein of VH-pIX and VL-pVII were expressed in the cell, and VH/VL displaying phage can be obtained from infection by a helper phage.

In the second selection step, the phage with high binding activity, which was obtained as above, was used to infect non-suppressing E.coli such as HB2151. Then through rescue by a helper phage, phages displaying VH as well as free VL fragment were obtained in the culture medium. In this case, because the amber codon was properly recognized as a stop codon by the host strain, all the phages obtained displayed only VH. A secretion signal of omp A sequence was placed at the upstream of VL, and the VL is secreted outside of the cell body in the same manner as phage particles.

### EXAMPLES

Examples will be presented below to illustrate the embodiments of the present invention further in detail.

### Example 1

### (a) Construction of a phagemid vector displaying gene encoding anti-lysozyme antibody HyHEL-10 on g7/g9

Necessary fragments were prepared by polymerase chain reaction (PCR), and they were ligated to prepare the desired phagemid vectors. The condition for PCR was as summarized in Table 1 below. The reaction condition of PCR described in Table 1 consisted of (94°C, 5 min) × 1, (94°C, 30 sec; 55°C, 30 sec; 72°C, 1 min) × 35, and (72°C, 8 min) × 1. All of the DNA polymerase used was KOD polymerase (2.5 units/100 µl, Toyobo, Osaka).

**TABLE 1**

| | back primer | forward primer | template |
|---|---|---|---|
| VH | M13RV | VH1for2X | pCANTAB-5E/HyHEL-10 |
| VL | VK2Back | Reverse SEQ | pCANTAB-5E/HyHEL-10 |
| g9-ompA(linker) | LinkBack | LinkFor | pHSG397/g9-ompA |
| VH-linker | M13RV | LinkFor | VH, linker |
| Linker-VL | LinkBack | Reverse SEQ | linker, VL |
| VH-linker-VL | M13RV | Reverse SEQ | VH-linker,linker-VL |

### Cloning of ompA-FLAG

The genes encoding E.coli ompA secretion signal sequence and FLAG tag sequence located at the N-terminal of VL were amplified by PCR using a plasmid pFLAG-ATS (Sigma-Aldrich) as a template. The primer sequences were designed such that the restriction sites of the restriction enzymes XbaI and SalI were inserted at the 5'-side and 3'-side of the amplified fragment, respectively. The amplified DNA was cloned into a cloning vector pHSG397 (TakaraBio, Otsu, Japan), and its sequence was confirmed using a fluorescent DNA sequencer (SQ-5500, Hitachi, Tokyo) and Thermosequenase sequencing kit (Amersham Bioscience, Tokyo)(this cloned vector is called pHSG397/ompA-FLAG hereinafter).

Back: ompXbaRV
5'-CGGGGTCGACTGTGCACTTTTGTCATCGTCGTCCTTGTAG-3'

Forward:   ompApaSalFR
5'-CGGGGTCGACTGTGCACTTTTGTCATCGTCGTCCTTGTAG-3'

### Cloning of gene IX, gene VII

A single strand DNA (ssDNA) was prepared from a helper phage M13KO7 (TakaraBio), and the sequences of gene VII and gene IX were amplified by PCR using the ssDNA as a template. The primers used were as follows and they are designed for the purpose to introduce KpnI and EcoRI sites for gene VII, XhoI and XbaI sites for gene IX at the 5'- and 3'-termini respectively, as the restriction sites of restriction enzymes.

Back: g7KpnRV
5'-CGGGGGTACCGCAGGTCGCGGATTTCGAC-3'

Forward:    g7EcoFR
5'-CGGGGAATTCTCATCTTTGACCCCCAGCG-3'

Back: g9XhoRV
5'-CGGGCTCGAGCGGTGGAGGCGGTTCAATGAGTGTTTTAGTGTATTCTTTC-3'

Forward: g9XbaFR
5'-CGGGTCTAGATCATGAGGAAGTTTCCATTAAAC-3'

The amplified gene VII fragment was digested with EcoRI and KpnI and then they are cloned into pBlueScript II KS+ (Toyobo). The amplified gene IX fragment was digested with XhoI and XbaI and then they are cloned into pHSG397/ompA-FLAG. Their sequences were confirmed (called pBS-g7 and pHSG397/ompA-g9 hereinafter).

### Preparation of linker

### Preparation of VH-linker-VL (HyHEL-10) fragment by overlap-extension PCR

The VH, VL and linker were amplified respectively by PCR using the following primers, and then each fragment of VH-linker and linker-VL were amplified by overlap-extension PCR. Further, by performing overlap-extension PCR on the VH-linker and linker-VL, a fragment of VH-linker-VL was obtained. Both termini of the fragment were digested with restriction enzymes NcoI and NotI. PCR amplification of linker:

Back: LinkBackX
5'-GGGACCACGGTCACCGTCTCGAGCGGTGGAGGCGGTTCAATG-3'

Forward:    LinkFor
5'-AGACTGGGTGAGCTCAATGTCCGTCGACTGTGCACTTTTGTC-3'

PCR amplification of VH (HyHEL-10):
Back:   M13RV

5'-CAGGAAACAGCTATGAC-3'
Forward:   VH1For2X

5’-GACGGTGACCGTGGTCCCTTGGCCCC-3’
PCR amplification of VL (HyHEL-10)

Back: Vk2Back
5'-GACATTGAGCTCACCCAGTCTCCA-3'

Forward: ReverseSEQ
Reference, 5'-GTAAAACGACGGCCAGT-3'

### Construction of phagemid vector pKS1

A phagemid vector pKS1 was constructed on pScFv3, which is a modified version of pK1 (Kristensen, P., and Winter, G. (1998) Folding & Design 3:321-328). PCR was performed using phagemid vector pIT2(13CG2) (de Wildt, R.M., Mundy, C.R., Gorick, B.D., and Tomlinson, I.M. (2000) Nat. Biotechnol., 18:989-994) encoding anti bovine serum albumin (BSA) single chain antibody (scFv) as a template with primer M13RV and MycAKpnFor (5'-CCGGGTACCTATGCGGCCCCATTCAGATC-3'), to amplify DNA fragments encoding anti BSA scFv and His-Myc tag. The DNA fragment, after dijested with SfiI, received end blunting with T4 DNA polymerase, then it was treated with KpnI and purified.

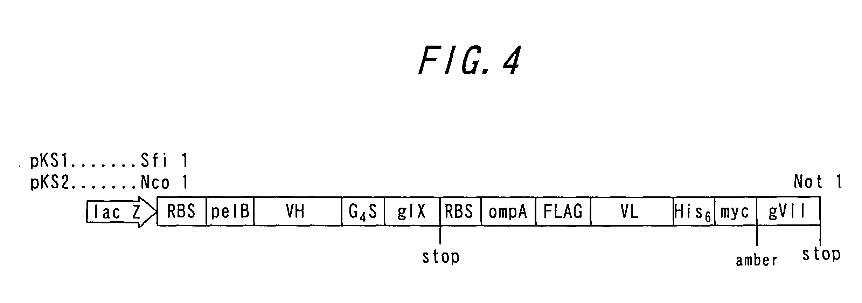

After phagemid vector pK1 was treated with PstI close to the 5' end of a gIII gene, blunted by T4 DNA polymerase and treated with KpnI, the resulting fragment was ligated with above-mentioned purified product. The resulting vector pScFv3 containing an anti-BSA scFv gene-His-Myc tag was treated with KpnI and EcoRI, to which fragment of gene VII obtained from pBS-g7 treated with KpnI and EcoRI was inserted (pScFv/g7). Subsequently, VH-linker-VL fragment treated with NcoI and NotI was inserted into pScFv/g7 to produce pKS1. The sequence of the phagemid vector pKS1 is shown in Fig. 4.

### Preparation of phages (VH/VL or VH displaying types)

The constructed phagemid pKS1 (ampicillin-resistant) was used to transform E.coli. The E. coli was grown at 37°C in 100 mL of 2TYAG medium (2TY + ampicillin, 100 µg/µL; glucose, 2%) until its growth reached a logarithmic phase. When O.D.600 = 0.5, to 10 mL of the culture medium, a helper phage M13KO7 was added at 20 equivalents of the amount of the bacterial cells. The culture was left at 37°C for 30 minutes, the supernatant containing glucose was removed, the cell pellet was resuspended in 100 mL of 2TYAK medium (2TY + ampicillin, 100 µg/mL; Km, 25 µg/mL), and the culture was incubated at 30°C overnight. The culture medium was then centrifuged at 4,000g for 15 minutes, the supernatant was recovered, 1/5 volume of 20% PEG6000/2.5M NaCl was added to it, the mixture was placed on ice for 1 hour, and centrifuged at 4,000g for 15 minutes, the precipitate was resuspended in 2 ml of TE (10 mM Tris-HCl; 1 mM EDTA; pH8.0). The suspension was centrifuged again and the supernatant was recovered to obtain a phage solution. The titer of the obtained phage was determined as described later, and the concentration of the phage particle was determined by colony forming ability (titer, cfu, or colony forming unit/mL).

### i) Suppressor strain

E.coli amber suppressor strains TG-1 and XL-1Blue were transformed by plasmid pKS1 to obtain a VH/VL displaying phage, displaying VH on pIX and VL on pVII.

### ii) Non-suppressor strain

A E.coli non-suppressing strain HB2151 was transformed by pKS1, and a phage solution containing phage displaying VH on pIX (without VL display), and soluble form VL fragments were obtained. Precipitation step using PEG/NaCl was omitted for this phage and the culture medium was used directly as it is.

### Determination of phage titer

The titer of the phage was determined as an index of the phage concentration. E.coli TG1 was cultivated on 2TY medium until logarithmic phase. One µL of the phage solution diluted 1000 fold with PBS was added to 100 µL to 1 mL of the E.coli culture. The resulting culture was kept at 37°C for 30 minutes to allow infection to occur. The culture was then diluted with 2TY medium, and 10 µL aliquot from each diluted solution was spotted on a 2TYAG plate. The plate was left overnight, the numbers of colonies formed in each spots were counted, and the number of colonies formed from 1 mL of original phage solution was calculated, and taken as an index of phage concentration.

### Phage ELISA

A 100 µL of solution containing 1-10 µg/mL solution of a primary antibody or an antigen was poured onto each well of a 96-well polystyrene microplate, and kept at 4°C overnight for immobilization. After solution was removed from each well, 200 µL of PBS containing 1% skim milk (MPBS) was poured, and the plate was left at room temperature for 2 hours for blocking. Subsequently, the plate was washed three times with PBS containing 0.1% Tween20 (PBST), and 100 µL of a phage solution (in MPBS) at 10¹¹ - 10¹² cfu/mL was added to it, and the plate was left at 37°C for 1 hour. The microplate was washed three times, 100 µL of an HRP-labeled anti-phage antibody (mouse anti-M13 HRP, Amersham Bioscience) diluted 5000 fold with MPBS was added to each well, and the plate was left at room temperature for 1 hour.

The microplate was washed three times with PBST, and an enzyme reaction solution prepared in advance (50 mL 100 mM sodium acetate, pH6.0; TMBZ (in DMSO) 500 µL; H₂O₂ 10 µL), was added to each well of the plate to initiate reaction. After reacted for 10 - 30 minutes in dark to allow the reaction to proceed, 50 µL of 3.2N H₂SO₄ was added to each well to terminate the reaction. The light absorption of each well was measured with a plate-reader (at 450 nm, and 650 nm for control).

### Open sandwich ELISA

The basic operation was the same as the phage ELISA described above. A primary antibody (αFLAG-M2 or amyc 9E10) for immobilization of tagged VL was immobilized onto a microplate, then blocking was performed, 100 µL of phage solution (in 1 % MPBS) (an antigen (HEL, 0.6 - 10 µg/mL) with known concentration had been mixed in advance) was added, and the plate was left at 37°C for 1 hour. The enzymatic activity of the HRP-labeled anti-phage antibody was determined by absorbance as described above for the phage ELISA.

### Confirmation of VL fragment display

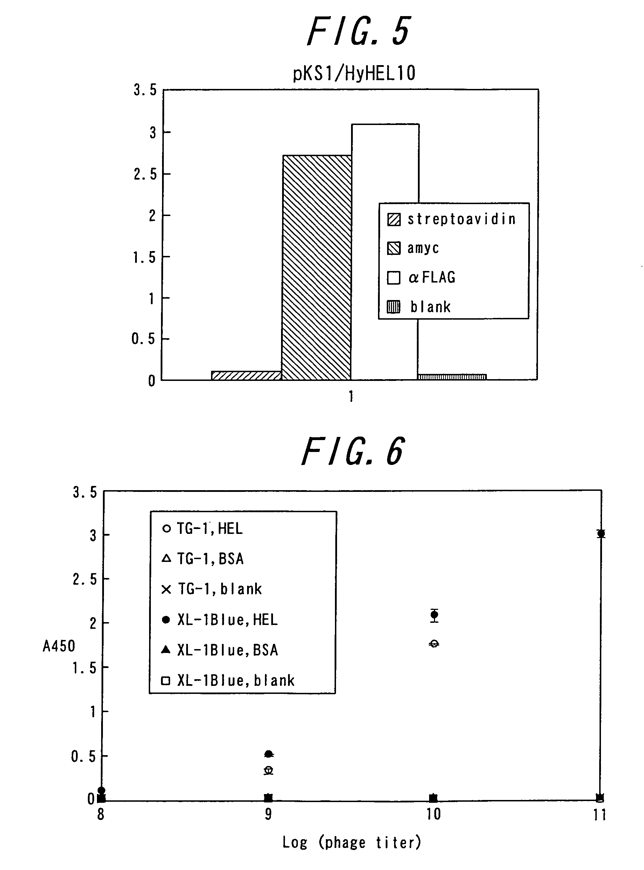

The VL fragment displayed by a phage was inserted with a myc tag on its N-terminus and a FLAG tag on its C-terminus. To confirm that the antibody fragment is displayed, phage ELISA was performed using antibodies specific to these tags. As a result, it was found at least the VL fragment was displayed sufficiently. The result of the experiment for the confirmation of display is shown in Fig. 5.

### Confirmation of antigen binding activity

To check whether VH and VL antibody fragments were sufficiently displayed on the phages, and whether the VH and VL fragments displayed on the phages had sufficient binding activities and specificity to the antigen, phage ELISA was performed using a HEL-immobilized microplate. As a result, specific binding to the antigen was confirmed when the concentration of the phage was 10⁹ (cfu/mL) or higher. This suggests that the VH fragment was displayed sufficiently. The result of confirmation on the binding activity to the antigen is shown in Fig. 6

### Comparison of scFv with split Fv

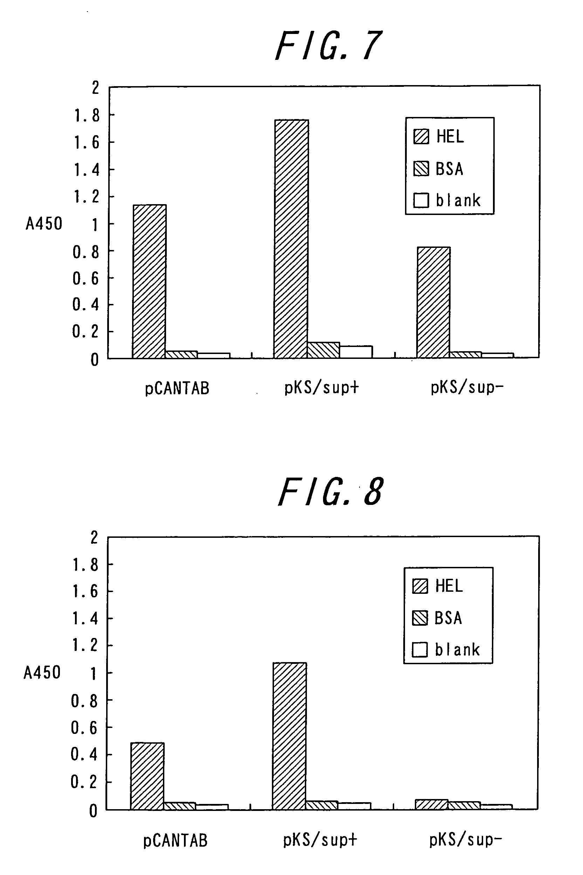

Phages displaying HyHEL-10 at scFv type and those displaying HyHEL-10 at split Fv type were compared for their antigen binding activity respectively, by subjecting them to ELISA using a microplate on which the antigen (HEL) had been immobilized. The split Fv type phages exhibited sufficient binding activities as high as the scFv displaying phages. This suggests phages with binding ability can be screened under the same selection conditions as used in conventional methods. Fig. 7 shows the result obtained at phage concentration of 10¹⁰ cfu/ml. Fig. 8 shows the result obtained at phage concentration of 10⁹ cfu/ml.

### Preparation of VH displaying phage

### Transformation of non-suppressing strain E.coli HB2151

The vector pKS1 constructed as above was used to transform a non-suppressor E.coli strain HB2151, and the transformant was rescued by helper phage M13KO7 to produce phages. The phages obtained displayed only VH, and VL was secreted to the culture medium apart from the phages. The titer of the medium was determined as described above, and used for the following experiments.

### Confirmation of display switch by non-suppressing strain

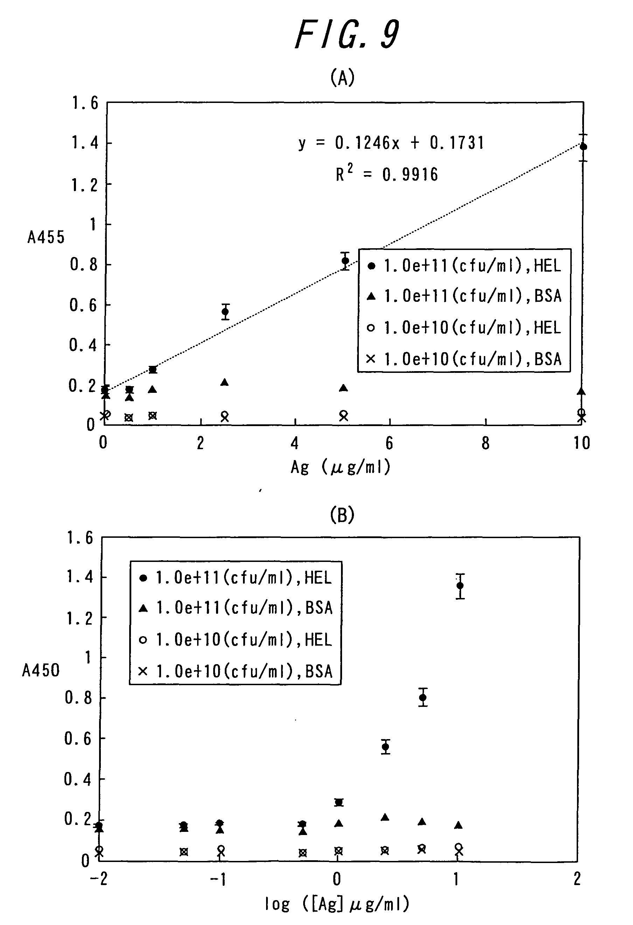

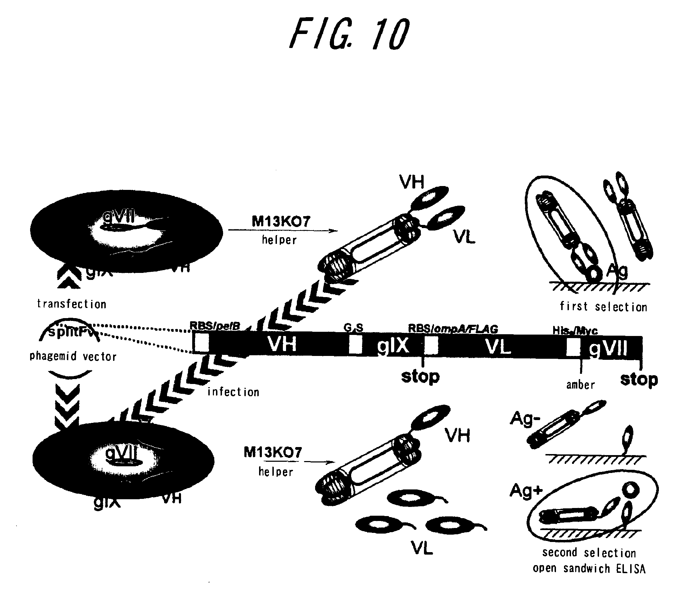

To confirm whether switching of display by the amber codon is sufficiently achieved by the non-suppressing inactive strain, open sandwich ELISA was performed using the culture medium directly at varying antigen (HEL) concentration in the range of 0 - 10 µg/mL. It was found that a dose-dependent increase in the signal intensity was observed at phage concentration of 1 × 10¹¹ (cfu/mL), therefore, it was assumed that display switch occurred sufficiently. The result also showed that a sufficient amount of VL was secreted. The results are shown in Fig. 9. Moreover, the mechanism underlying display switch in this experimental system is shown in Fig. 10.

In an actual screening, clones were selected from a phage library comprising VH/VL displaying phages exhibiting high affinity to an antigen of interest, and each of the selected clones were cultured on a 96 well plate to produce phages. It was revealed that the phage titer of the culture medium sampled from any wells on the 96 well plate was approximately in the order of 10¹² and there was no significant difference observed. Thus it was also suggested that open sandwich ELISA can be directly utilized for the screening.

### Preparation of phagemid vector pKS2

The pKS1 prepared as above was disadvantageous in that it was difficult to insert a gene fragment having an NcoI cleavage site inside of the fragment, because it contains only 6-base NcoI recognition site at upstream of the VH for the cloning of an antibody. To improve this defect, another vector pKS2 was prepared; and as to this vector, 8-base recognizing enzyme SfiI in addition to NcoI could be utilized for cloning.

Specifically, pKS1-(HyHEL-10) was cleaved by NcoI and EcoRI, and a resulting 0.8 kb insertion fragment was inserted via ligation into a phagemid vector pCantab5E (Amersham Bioscience), which had been digested with NcoI and EcoRI.

### Preparation of phagemid vector incorporated with a terminator

Depending on the antibody gene to be inserted, it was observed that expression of a fusion protein prior to induction of the expression brought harmful effects on the growth of the host E.coli, which led to the phenomenon of deletion of the inserted gene at high probability. To prevent the phenomenon, a translation termination sequence derived from glutamine permease operon (terminator tHP, Nohno, T. et al., Mol. Gen. Genet. (1986) 205:260-269) was inserted at upstream of the translation initiation signal and at downstream of the fusion protein gene. It was expected that the background expression of the fusion protein from mRNA, whose transcription is initiated from the promoter-like sequence existing in the phagemid vector, would be reduced when protein expression is not induced.

A phagemid vector pKS2 was digested at SapI site upstream of Lac promoter. The following four oligonucleotides were phosphorlylated and annealed to prepare a terminator gene, which was inserted into the cleaved site.

tHP1 5'-AGC GGT ACC CGA TAA AAG CGG CTT CCT GAC-3'

tHP2 5'-AGG AGG CCG TTT TGT TTT GCA GCC CAC CTC-3'

tHP3 5’-GCT GAG GTG GGC TGC AAA ACA AAA CGG CCT-3’

tHP4 5'-CCT GTC AGG AAG CCG CTT TTA TCG GGT ACC-3'

The recombinant phagemid was confirmed for the nucleotide sequence that it was as designed (pKS2T).

Then, a phagemid pKS2T was digested at an EcoRI site existing downstream of the fusion protein gene. The following two primers tHP7 and tHP8, as well as tHP2 and tHP3 were annealed to prepare a terminator gene, which was inserted into the cleaved site.

tHP7 5'-AAT TGG TAC CCG ATA AAA GCG GCT TCC TGA C-3'

tHP8 5’-AAT TGA GGT GGG CTG CAA AAC AAA ACG GCC T-3’

Determination of the nucleotide sequence of the phagemid with the above insert revealed that two terminators linked in tandem were inserted at the EcoRI site. The phage was named pKST2.

### Selection of antibodies suitable for open sandwich method

A study was performed to see whether suitability for the open sandwich method could be evaluated on a given antibody using this system. The anti hen egg-white lysozyme antibodies HyHEL-10 and D1.3 were used as the antibody genes. It had been known that D1.3 exhibits strong VH-VL interaction, regardless of the presence/absence of the antigen. In the same manner as described above, PCR was performed with the D1.3 Fv gene as a template to produce a split Fv gene. The gene was inserted into pKST2, which was then used to transform non-suppressing strain HB2151. Helper phage M13K07 was used to prepare culture medium containing phages displaying VH and secreted VL.

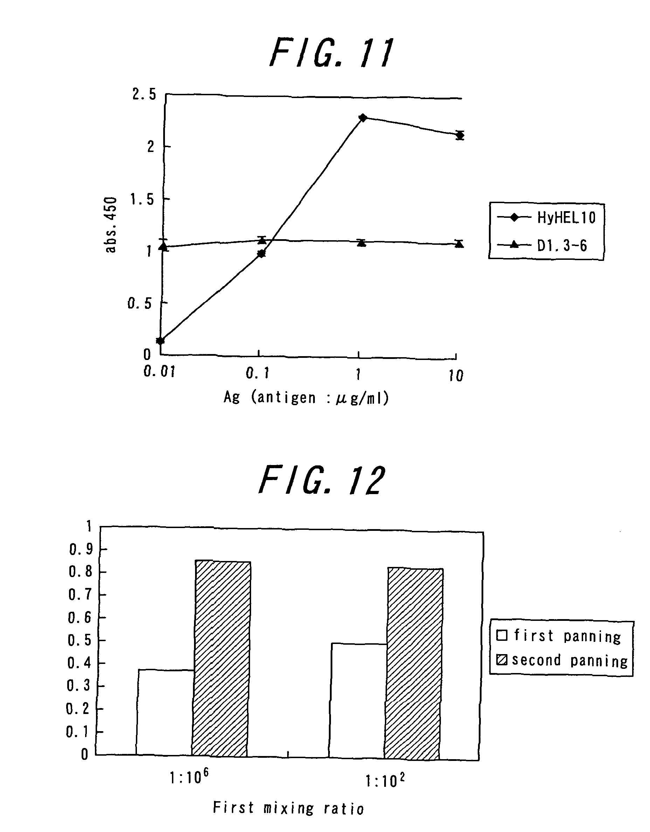

The medium and the antigen were poured on a microplate on which anti-FLAG tag antibody had been immobilized. As a result, when the dependency of phage binding to antigen concentration was investigated, in the case of D1.3, it was found that the amount of phage immobilized via VL fragment did not show considerable change. In contrast, when HyHEL-10 was used, the amount of phages immobilized via the VL fragment markedly increased depending on the lysozyme concentration in the sample. From this, it was concluded that this system is available as a simplified screening method for antibodies suitable for open sandwich method. The results are shown in Fig. 11.

### Model panning

To demonstrate that phages displaying antibodies exhibiting specific binding can be selected by this system, using the panning method, an attempt was made to concentrate phage antibodies having specificity of interest from a mixture containing two kinds of anti-phage antibodies having different specificities.

The used phagemid included pKST2-H10 (H10) capable of displaying split Fv encoding HyHEL-10, and pKST2-31IJ3 (IJ3) capable of displaying anti-fluorescein split Fv. These were used to transform E.coli TG-1 infected by a helper phage M13KO7, and split Fv displaying phages were produced. These were used to prepare phage solution mixed to give the titer of H10:IJ3 = 10⁵:10¹¹ or 10⁹:10¹¹ in 100 µl of MPBS. The phage solution was poured onto a microplate on which hen egg-white lysozyme (HEL) had been immobilized. The plate was then kept at 37°C for 1 hour, and washed two times with PBS-T and two times with PBS. A 100 µL of 0.2 M glycine-HCl (pH 2.2), 1 mg/ml bovine serum albumin was poured onto the plate to elute phages, which were then neutralized with 6 µL of 2 M Tris. The resulting phage solution was used to infect E.coli TG-1 at a logarithmic growth phase and allowed to infect (first panning). The culture was inoculated onto a YTAG plate, which was then left at 37°C overnight. Four to eight colonies were recovered and cultivated respectively to produce phages (monoclonal phages). Along with them, the remaining colonies were collected, and phages were prepared from them in a same manner. They were used again to perform panning using a microplate on which HEL had been immobilized. Monoclonal phages were prepared from 48 clones, and each of them were assayed for their binding ability to HEL by ELISA.

As a result, it was found that the first panning brought about significant enrichment of HEL binding clones at both mixing ratios of 1:10⁶ or of 1:10², and the ratio of bound clones in terms of total was 37.5% and 50% respectively. And after the second panning, more than 80% of the clones became positive for both mixing ratios (Fig. 12).

### EXAMPLE 2

HyHEL-10 is suitable for open sandwich method while D1.3 is not suitable for its strong VH/VL interaction, though both are antibodies toward HEL. If amino acid residues involved in the VH/VL interaction were identified, the residues may replaced with the corresponding amino acid residues of an antibody applicable for the open sandwich method, thereby the open sandwich method could be applied to various antibodies. In addition, no systematic study has been made on the relationship between the VH/VL interaction and its binding ability to an antigen, or the sequence of the framework region of the antibody. The second framework region (FR2) locating at the VH/VL interface was noticed, and studies were performed on the following; in the case the residues in FR2 of VH and VL of HyHEL-10 are replaced by those derived from D1.3, whether or not the open sandwich method can be still applied for the resulting product, and how the VH/VL interaction would be altered from that of corresponding wild type (WT) strains. During the study, the ratio of clones applicable to the open sandwich method was evaluated. Moreover, study was also performed to identify the residues indispensable to satisfy the two conditions required for the open sandwich method.

### Preparation of split Fv fragment

PCR was performed using HyHEL-10 as a template by the method as described below. Mixed bases are inserted into the primers and each residue in FR2 region was modified to encode either HyHEL-10 type or D1.3 type. Thus, a DNA fragment encoding from the N-terminal of VH to the 55th Gly (H55) located at CDR2 region of VL (to be referred to as VH FR2) was amplified (MH2BackSfi was used as a reverse primer and H10VHframe2 was used as a forward primer). Moreover, a DNA fragment encoding from the 30th Gly (L30) located at CDR1 region of VL to the N-terminal of VL gVII was amplified (to be referred to as VL FR2)(H10VLframe2 was used as a reverse primer and g7EcoFR was used as a forward primer).

For the template of PCR reaction, a phagemid (pKST2 HyHEL-10A) was used, which carried split Fv fragment of HyHEL-10 where the FLAG tag of pKST2 was moved from the N-terminus of VL to the C-terminus of VH. Similarly a linker necessary to convert the two DNA fragments to split Fv fragments was also amplified. pKST2 HyHEL-10A was also used as a template in this case. A DNA fragment from the 49th Gly (H49) located at CDR2 region of VH to the C-terminal of VH, FLAG, gIX, OmpA, to the 37th Gln (L39) located at CDR1 region of VL was amplified (linker (FR2))(H10linkRV was used as a reverse primer and H10linkFR was used as a forward primer).
In either case, Ex Taq (Takara Bio) was used as DNA polymerase. The reaction started from (94°C, 5 min), then 35 cycles of (94°C, 30 sec; 55°C, 30 sec; 72°C, 1 min), followed by (72°C, 5 min), and ended at 4°C.

Next, VH FR2, VL FR2 and linker (FR2) were assembled into one DNA fragment by overlap extension PCR. First, in the absence of primers, VH FR2, VL FR2 and linker (FR2) were added into a PCR solution, and reaction started from (94°C, 5 min), then 7 cycles of (94°C, 30 sec; 55°C, 30 sec; 72°C, 1 min), and ended at 4°C. Then, using MH2BackSfi as a reverse primer and g7EcoFR as a forward primer, reaction was performed 30 cycles of (94°C, 30 sec; 55°C, 30 sec; 72°C, 1 min), and ended at 4°C. The used polymerase was Ex Taq.

H10VHframe2:
5'-
ACCACTGTAGCTTACGTACCCCAWSYACTCCAGACSKTTACCTGG
ARRTTKACGAAYCCAGCTCCAATAATCACTGGT-3'

H10VLframe2:
5'-
GGCAACAACCTACACTGGTATCAACAAAAAYMGSRCRAATCTCCT
CRGCTCCTGRTCWAKTATGCTTCCCAGTCCATCTCT-3'

H10linkRV:
5'-GGGTACGTAAGCTACAGTG-3'

H10linkFR:
5'-GATACCAGTGTAGGTTG-3'
(K=G or T, M=A or C, R=A or G, Y=C or T, S=C or G, W=A or T)

### Confirmation of the randomization of FR2 region

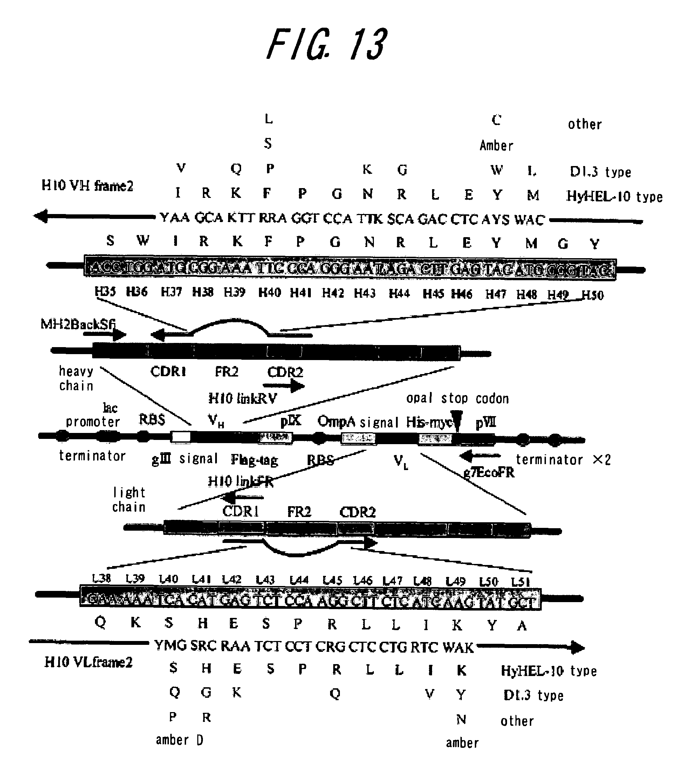

The split Fv fragment thus amplified was digested with NcoI and NotI, inserted into a vector pKST2 treated with NcoI and NotI. The resulting vector was used to transform E.coli TG-1^{sup+}. The cells were applied to a YTAG plate (Ap, 100 µg/mL; glucose, 1%), and the plate was incubated at 30°C overnight. Colony PCR was performed on the resultant colonies. The insertion of split Fv fragment was confirmed on the clones, such clones were inoculated from a replica plate, and the culture was incubated at 37°C overnight with shaking. Plasmid was purified from the resultant bacterial culture by alkaline SDS method. The sequence was confirmed with an ABI PRISM 3100 genetic analyzer (Applied Biosystems) and BigDye Terminator Cycle Sequencing Kit (Applied Biosystems). The used primers were M13RV, and OmpARV (reverse primer complimentary to the partial sequence of OmpA signal). To confirm phage display of VH and VL, then to confirm the display of functional Fv, anti-FLAG M2, 9E10 and antigen HEL, were immobilized respectively and phage ELISA was performed along with blank wells. Fig. 13 shows a schematic diagram showing production of split Fv fragments wherein respective residues in FR2 region of HyHEL-10 are modified to HyHEL-10 type or D1.3 type.

OmpARV:
5'-ACAGCTATCGCGATTGCAGTG-3'

### Preparation of library

The split Fv fragments as described above were prepared at a large scale. Then 2.13 pmol of the vector (pKST2) and 21.3 pmol of DNA fragments to be inserted (split Fv fragments having mutations introduced into FR2 region of HyHEL-10) were mixed, and 1/2 vol. of Ligation High (Toyobo) was added. The mixture was left at 16°C for 2 hours to cause ligation. Desalting by ethanol precipitation was performed prior to transformation by electroporation. Specifically, 3 M sodium acetate corresponding to 1/10 vol. of the total ligation solution, 2 µL of Pellet Paint Co-Precipitant (Merck, Tokyo), and 2 vol. of 100% ethanol were added to the ligation solution, and the mixture was stirred with a vortex mixer, and the mixture was allowed to left at room temperature for 2 minutes. Then the mixture was centrifuged at 15,000 rpm at room temperature for 5 minutes, and the supernatant was discarded. The precipitate was rinsed with 70% ethanol, then 100% ethanol, and centrifuged at 15,000 rpm at room temperature for 5 minutes to remove the supernatant. The precipitate, after being dried by centrifugation, was dissolved into 20 µL of milli-Q water, and the solution was divided into two equal aliquots.

The 10 µL of the desalted DNA solution and 100 µL of E. coli (TG-1^{sup+}) were mixed, and the mixture was transferred to an electropolation cuvette to which electric voltage was applied with Easyject (EquiBio). Then, 900 µL of 2YT liquid medium was transferred to the cuvette, the mixture was suspended and transferred to a microtube, subsequently incubated at 37°C for 30 minutes. The same procedure was performed on the remaining 10 µL of the desalted DNA solution. After incubation, 1 µL of the culture was diluted with 99 µL of 2YT liquid medium, and 10 µL of the diluted solution was further diluted 10 folds by adding 90 µL of 2YT liquid medium. The same operation was repeated twice. A 10 µL of each diluted solution was spotted on a YTAG plate (Ap, 100 µg/mL; glucose, 1%), and the culture was incubated at 30°C overnight. The number of colonies grown at each spot was counted, and the size of the library was calculated. The remaining culture was applied to a large YTAG (Ap, 100 µg/mL; glucose, 1%) plate (Biotray) (Sumitomo Bakelite), and incubated at 30°C overnight.

### Preparation of phage library

A 5 ml of 2YT liquid medium was poured onto a bio-tray carrying grown colonies, and the colonies were recovered using a conradi stick. To the recovered colony, 1/2 vol. of 50% glycerol was added to produce a glycerol stock and the stock was stored at -80°C. A 50 µL of the bacterial solution thus recovered was added to 100 mL of 2YT liquid medium (Ap, 100 µg/mL; glucose, 1%), and the culture was incubated at 37ºC with shaking. When O.D.600 reached to 0.5, 20 equivalents (with respect to the amount of bacterial cells) of the helper phage M13K07 were added to 10 mL of the culture medium, and the culture was left at 37°C for 30 minutes. Then, the culture was centrifuged at 2,000 rpm for 15 minutes, the supernatant was discarded, and the precipitate was re-suspended into 5 mL of 2YT liquid medium (Ap, 100 µg/mL; Km, 50 µg/mL). The suspension was transferred to a conical flask (attached with baffle) filled with 45 mL of 2YT liquid medium (Ap, 100 µg/mL; Km, 50 µg/mL). The culture was incubated at 30°C for 20 hours with shaking at 250 rpm/min. After incubation, the culture was centrifuged at 6,500g for 10 minutes, and the supernatant was recovered. To the supernatant, 1/5 vol. of 20% PEG6000/2.5M NaCl was added, and the mixture was left on ice for 1 hour. Later, the culture was centrifuged at 6,500g, 4°C, for 30 minutes, then phage obtained as a precipitate was re-suspended with 1 mL of TE (10 mM Tris; 1 mM EDTA; pH8.0), and the suspension was again centrifuged at 15,000 rpm, 4°C, for 20 minutes, and the supernatant was recovered to produce a phage library.

### Biopanning and monoclonal phage ELISA

Biopanning was repeated once or twice with HEL as an antigen. To obtain clones that specifically bind to HEL, from the population of phages selected by biopanning, monoclonal phage ELISA was performed. For the total of 1536 clones, ELISA was performed using the supernatants of phage culture, and clones were selected according to the following criteria; the absorbance of the well immobilized with HEL should be 0.3 or higher, and absorbance should be five times or more compared with that of blank. For 72 clones thus selected, it was investigated whether their binding to HEL was specific or not.

Specifically, during the preparation of the library, about 7 × 10⁷ colonies were obtained. From them, clones revealed to have no insert by colony PCR and clones revealed to have excess or deficiency in gene were subtracted. Then, the diversity of the resulting library was estimated to be 5 × 10⁷. The library was subjected to a round of biopanning to obtain 1536 clones. Phage culture medium prepared on a 96-well plate was used to perform ELISA. The clones were selected according to the criteria; the absorbance of the wells immobilized with HEL should be 0.3 or higher, and the absorbance should be five times or more compared with that of blank. For 72 clones thus selected, phages were re-prepared at 5 mL scale, and investigation was performed using ELISA as to whether their binding to HEL was specific or not, and as to whether they displayed VH or VL.

### Specificity of phage clones to HEL

Of the clones that were revealed to have high binding ability to HEL by monoclonal phage ELISA, the clones were further confirmed for the specificity to antigen. For each clone, a glycerol stock was prepared, and an aliquot therefrom was inoculated, phage display was performed at 5 mL scale, and then phage ELISA was performed. To confirm the display of Fv, HEL immobilized and blank wells, and to confirm that of VH and VL, wells immobilized with anti FLAG M2 and 9E10, respectively, were prepared. As a result, obtained clones displayed both VH and VL and bound to HEL with sufficient selectivity. For each of the clones, an aliquot from their glycerol stock was inoculated and incubated with shaking at 37°C overnight. Plasmids were purified from the bacterial culture by alkaline SDS method. The sequence was confirmed with an ABI PRISM 3100 genetic analyzer and BigDye Terminator Cycle Sequencing Kit with primers M13RV and OmpARV.

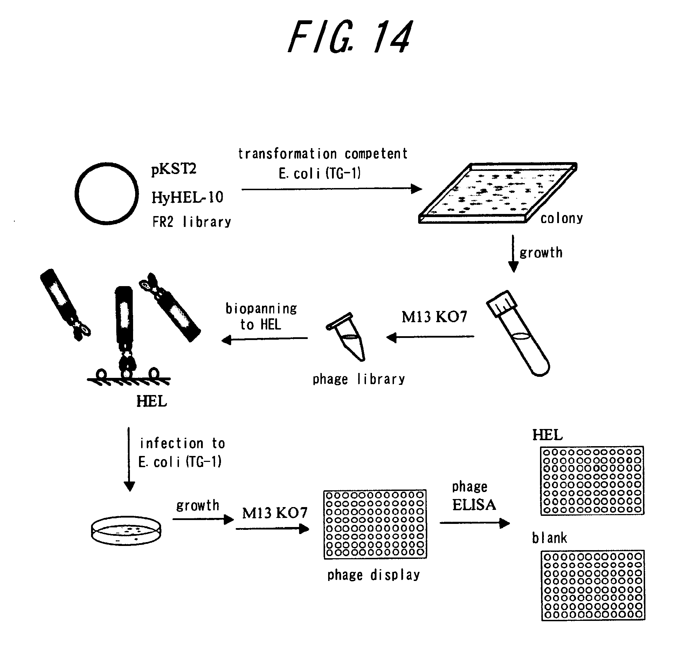

Clones were selected according to the criteria; the absorbance of the wells immobilized with anti FLAG M2, 9E10 should be sufficiently high enough to confirm the display of both VH and VL, and the absorbance of the well immobilized with HEL should be eight times or more than that of blank, enough to confirm that the binding is specific. As a result 64 clones were obtained. Since mutations were introduced into the framework region that is intrincially conservative, it might have generated considerable clones with non-specific binding. For these selected clones, the amino acid sequences were determined for the displayed VH and VL. Based on the results, clones substituted to the amber codon, and clones having amino acid sequences identical to WT with respect to both of VH and VL were excluded from the study. Then the remaining 36 clones were further studied. Fig. 14 shows a flow chart of the procedures from preparation of a phage library, to selection of clones exhibiting high binding ability to HEL.

For the 36 clones, the titer of each phage solution was adjusted to be 2.5 × 10⁸, 1 × 10⁹, or 4 × 10⁹ (cfu/ml), and ELISA was performed on each clones in respect to HEL or blank. All the clones exhibited specific binding to HEL, but the binding abilities varied wide; some was weaker than WT, some were equivalent to WT and some were stronger than WT. The value calculated as follows are used as index of binding ability to HEL; ELISA was performed at 1 × 10⁹ cfu/ml, and the absorbance was measured on wells immobilized with HEL from which the absorbance of blank was subtracted, and the subtracted value was divided by corresponding value from WT.

### Measurement of VH/VL interaction by open sandwich ELISA

For the 36 clones that exhibited sufficientlypecific binding to HEL without substitution by amber codons, phages were allowed to be infected by HB2151^{sup-} to produce supernatants containing phages displaying only VH and soluble VL fragments. The supernatant was applied to a well on which 9E10 was immobilized, the 9E10 being an antibody specific to myc tag (tag attached to VL), and open sandwich ELISA was performed with various concentrations of the antigen (HEL = 0, 0.1, 1, 10 µg/mL). A blank well was also prepared to determine the absorption due to non-specific binding of the antibody to the plate. The other operations were performed similarly as the open sandwich ELISA as described above in Example 1.

To determine whether a given clone is suitable for the open sandwich method, the value calculated as follows was adopted as an index; namely, the absorbance at HEL = 10 µg/mL was divided by the absorbance at HEL = 0 µg/mL. The clones were classified according to the index value: when the calculated value was lower than 1.2, the clone was determined to be not suitable for measurement by the open sandwich method, and when the calculated value was 1.2 or higher, the clone was judged to be suitable for measurement by the open sandwich method. As a result, among 14 clones determined to be suitable for the open sandwich method, in 13 clones the 39th amino acid residue (H39) of VH was K, whereas among 22 clones determined to be not suitable for the open sandwich method, in 20 clones the corresponding amino acid residue was Q.

Then, using the HB2151^{sup-}, and supernatant containing phages displaying only VH and soluble VL fragments, the VH/VL interaction was determined in the absence of the antigen. The 9E10, an antibody to a myc tag (the tag for VL), was left on a plate at 4°C overnight for immobilization. A 200 µL of 2% MPBS was added on the plate and left at room temperature for 2 hours. After blocking, the plate was washed three times with PBS-Tween (0.1% Tween20). To the plate, 100 µL of a mixture comprising 10 µL of the culture medium and 90 µL of 1% MPBS was added, and the plate was left at 37°C for 1 hour. The plate was washed three times with PBS-Tween (0.1% Tween20), 100 µL of murine HRP-labeled anti-phage antibody diluted 5000 fold was added to each well, and the plate was left at room temperature for 1 hour. The plate was washed three times with PBS-Tween (0.1% Tween20), and incubated for the enzymatic reaction.

The absorbance of the well immobilized with 9E10 was measured from which the absorbance of the blank was subtracted. Then the value thus calculated was adopted as an index to evaluate the VH/VL interaction. The test clones were arranged in the ascending order of the calculated value, and the sequences of amino acid residues in the FR2 region were compared among them. The clones were classified according to the calculated value: when the value was 0.5 or higher, the clone was determined to have a strong VH/VL interaction, and when the value was less than 0.5, the clone was determined to have a weak VH/VL interaction. Even in this case, the amino acid residue of H39 exerted a significant effect on the result. Among the 19 clones which were found to have a strong VH/VL interaction, H39 was Q in 17 clones, which is D1.3 type. In contrast, among the 17 clones which were found to have a weak VH/VL interaction, H39 was K in 13 clones, which is HyHEL-10 type. It was suggested from the above experiments that the amino acid residue at the 39th (H39) position in VH is deeply involved in the propriety for determination by the open sandwich method, and of the strength of its VH/VL interaction.

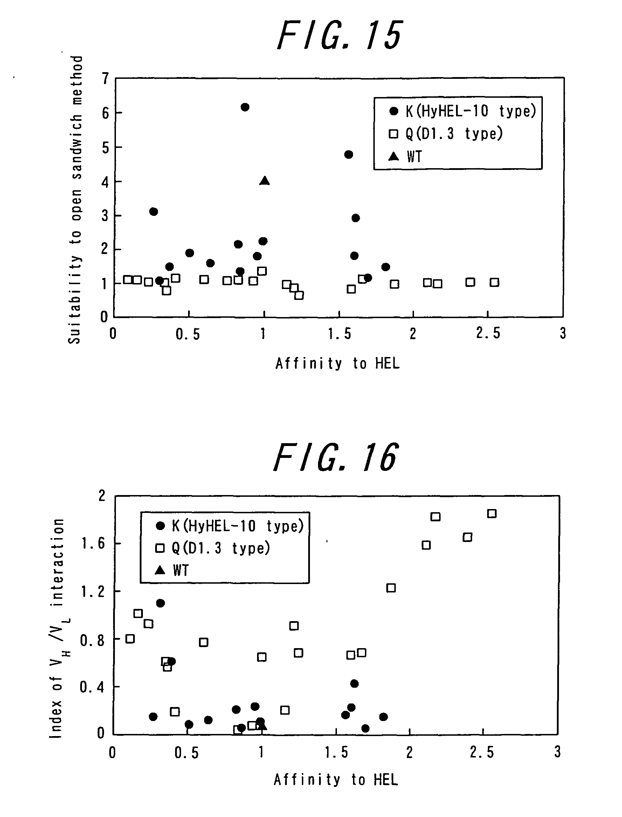

The relationships among their binding abilities to HEL, suitability for the open sandwich method, and the strength of VH/VL interaction were plotted. The relationship between the binding activities of the clones to HEL and their suitability for the open sandwich method is shown in Fig. 15. The relationship between the binding activities of the clones to HEL and their VH/VL interaction is shown in Fig. 16. In Figs. 15 and 16, solid circles represent the results from HyHEL-10 type antibodies (H39 = K), open squares represent the results from D1.3 type antibodies (H39 = Q), and solid triangles represent the results from wild type (WT). The results from the 36 clones were plotted, and they were classified by the amino acid at H39, namely whether H39 was K or Q.

The results also confirmed the above observation: the 39th amino acid residue of VH is deeply involved in the strength of VH/VL interaction of an antibody and its suitability for the open sandwich method. In the HyHEL-10 type antibody having K at H39, it has a weak VH/VL interaction, and is suitable for the open sandwich method. On the other hand, in the D1.3 type antibody having Q at H39, it has a strong VH/VL interaction, and is likely to be unsuited for the open sandwich method. The reason is likely to be due to whether hydrogen bond can be formed with Q at the 38th position (L38) of VL or not.

Actually, as to the HyHEL-10 type antibody in which H39 is K and the D1.3 type antibody in which H39 is Q, their three-dimensional structures were studied on the region proximal to H39. Then it was found that in the HyHEL-10 type antibody, no hydrogen bond is formed between K at H39 and Q at L38, while in the D1.3 antibody, double hydrogen bonds are formed between Q at H39 and Q at L38. Then in the case the antibody has K at H39, it fails to form a hydrogen bond with Q at L38, therefore, the VH/VL interaction will be weak. On the other hand, if the antibody has Q at H39, it will form double hydrogen bonds with Q at L38, therefore, the VH/VL interaction will be strong.

From above results it was demonstrated that selection of randomized antibody Fv library could be performed using the split Fv system. Then a large number of clones having higher affinities to an antigen than the wild type antibody, or those suitable for the open sandwich method could be obtained. In addition, from the above results, the characteristics of the antibodies suitable for the open sandwich method were also demonstrated.

The present invention provides a novel method for determining the interaction between VH and VL fragments of the variable region of an antibody. The invented method can be widely applied for the detection of interaction between proteins. According to the invented method, a phagemid vector containing the amber codon is used to transform the amber suppressor strain of E.coli, to produce phages on which both of VH and VL are displayed. On the other hand, when the amber non-suppressing strain of E.coli is transformed to produce phages, for the existence of amber codon, only the VH fragment is displayed on the phage particles whereas the VL fragment is secreted into the culture medium, thereby display switch can be achieved. It is possible to determine the interaction between the VH fragment and the VL fragment, by immobilizing the VL fragment secreted into the culture medium on a solid support, and quantifying its interaction with the VH fragment displayed on phages.

## Claims

1. A vector containing at least following five DNA sequences:
(1) a DNA sequence encoding one protein or its fragment; (2) a DNA sequence encoding a protein for displaying said one protein or its fragment on a phage; (3) a DNA sequence encoding another protein or its fragment; (4) a stop codon that enables display switch by a host strain; and (5) a DNA sequence encoding a protein for displaying said another protein or its fragment on the phage, the vector having a structure comprising these 5 DNA sequences in the order of (1), (2), (3), (4) and (5) or (3), (4), (5), (1) and (2) in the 5'-3' direction of the vector, by the presence of the stop codon that enables display switch by said host strain, when the vector is introduced into a suppressor-mutant host strain, the vector provides a two-protein displaying phage on which both of said one protein or its fragment and said another protein or its fragment are displayed, and when the vector is introduced into a non-suppressing host strain, the vector provides a one-protein displaying phage on which only said one protein or its fragment is displayed and said another protein or its fragment is secreted into the culture medium.

2. The vector as described in Claim 1 wherein said one protein or its fragment is a VH fragment of the variable region of an antibody, and said another protein or its fragment is a VL fragment of the variable region of an antibody.

3. The vector as described in Claim 1 wherein said one protein or its fragment is a VL fragment of the variable region of an antibody, and said another protein or its fragment is a VH fragment of the variable region of an antibody.

4. The vector as described in any one of Claims 1 to 3 wherein the vector is a phage vector or phagemid vector of E.coli.

5. The vector as described in any one of Claims 1 to 4 wherein said DNA sequence encoding a protein for displaying said one protein or its fragment on the phage is a DNA sequence encoding pIX protein of a filamentous phage, and said DNA sequence encoding a protein for displaying said another protein or its fragment on the phage is a DNA sequence encoding pVII protein of a filamentous phage.

6. The vector as described in any one of Claims 1 to 4 wherein said DNA sequence encoding a protein for displaying said one protein or its fragment on the phage is a DNA sequence encoding pVII protein of a filamentous phage, and said DNA sequence encoding a protein for displaying said another protein or its fragment on the phage is a DNA sequence encoding pIX protein of a filamentous phage.

7. A vector as described in any one of Claims 1 to 6 wherein said stop codon that enables display switch by a host is an amber codon.

8. A method for determining interaction between one protein or its fragment and another protein or its fragment, the method comprising the steps of:
(1) transforming a non-suppressing host strain by using the vector according any one of Claims 1 to 7, thereby obtaining one-protein displaying phage on which only said one protein or its fragment is displayed, and a culture medium containing said another protein or its fragment being secreted from said non-suppressing host strain;
(2) immobilizing said another protein or its fragment in the supernatant on an appropriate support;
(3) reacting said another protein or its fragment immobilized on the support with said one protein or its fragment displayed on the one-protein displaying phage, thereby said one protein or its fragment is bound to said another protein or its fragment; and
(4) determining the amount of immobilized phages by an immunoassay using a labeled anti-phage antibody, thereby evaluating the binding ability between said one protein or its fragment and said another protein or its fragment.

9. The method as described in Claim 8 wherein said one protein or its fragment is a VH fragment of the variable region of an antibody, and said another protein or its fragment is a VL fragment of the variable region of an antibody.

10. The method as described in Claim 8 wherein said one protein or its fragment is a VL fragment of the variable region of an antibody, and said another protein or its fragment is a VH fragment of the variable region of an antibody.

11. The method as described in any one of Claims 8 to 10 wherein the vector is a phage vector or phagemid vector of E.coli.

12. The method as described in any one of Claims 8 to 11 wherein said DNA sequence encoding a protein for displaying said one protein or its fragment on the phage is a DNA sequence encoding pIX protein of a filamentous phage, and said DNA sequence encoding a protein for displaying said another protein or its fragment on the phage is a DNA sequence encoding pVII protein of a filamentous phage.

13. The method as described in any one of Claims 8 to 11 wherein said DNA sequence encoding a protein for displaying said one protein or its fragment on the phage is a DNA sequence encoding pVII protein of a filamentous phage, and said DNA sequence encoding a protein for displaying said another protein or its fragment on the phage is a DNA sequence encoding pIX protein of a filamentous phage.

14. The method as described in any one of Claims 8 to 13 wherein said stop codon that enables display switch by a host is an amber codon.

15. The method as described in any one of Claims 8 to 14 wherein said suppressor-mutant host strain is an E.coli amber-suppressor strain.

16. The method as described in Claim 15 wherein said E.coli amber-suppressor strain is a strain TG1 of E. coli, and said non-suppressor host strain is E.coli strain HB2151.

17. A method for obtaining a variable region of an antibody in which interaction between a VH fragment and a VL fragment alters by the presence of an antigen, the method comprising the steps of:
(1) transforming a suppressor-mutant host strain by using a vector as described in Claim 2 to obtaining a VH/VL displaying phage, on which both of the VH and VL fragments are displayed when said vector is introduced into the suppressor-mutant host strain;
(2) confirming the binding ability between the VH/VL displaying phage obtained in the step (1) and an antigen;
(3) transforming a non-suppressing host strain by using said vector as described in claim 2 whose ability to provide a VH/VL displaying phage is confirmed in the step (2), or transfecting a non-suppressing host strain with the VH/VL displaying phage obtained in the step (1), thereby obtaining a VH displaying phage on which only the VH fragment is displayed and a culture medium containing the VL fragment secreted by the non-suppressing host strain;
(4) immobilizing said VL fragment in the supernatant onto an appropriate support;
(5) reacting said VL fragment immobilized on the support with said VH fragment displayed on the phage in the presence or absence of an antigen;
(6) determining the amount of immobilized phages by an immunoassay using a labeled anti-phage antibody, thereby evaluating the binding ability between said VH fragment and said VL fragment; and
(7) determining that an antibody variable region in which interaction between the VH fragment and the VL fragment significantly alters in the presence of an antigen is obtained, provided that the binding ability between said VH fragment and said VL fragment in the presence of an antigen is two times or more than the binding ability between the VH fragment and the VL fragment in the absence of the antigen.

18. A method for obtaining a variable region of an antibody in which interaction between a VH fragment and a VL fragment alters by the presence of an antigen, the method comprising the steps of:
(1) transforming a suppressor-mutant host strain by using a vector as described in Claim 3 to obtaining a VH/VL displaying phage, on which both of the VH and VL fragments are displayed when said vector is introduced into the suppressor-mutant host strain;
(2) confirming the binding ability between the VH/VL displaying phage obtained in the step (1) and an antigen;
(3) transforming a non-suppressing host strain by using said vector as described in claim 3 whose ability to provide a VH/VL displaying phage is confirmed in the step (2), or transfecting a non-suppressing host strain with the VH/VL displaying phage obtained in the step (1), thereby obtaining a VL displaying phage on which only the VL fragment is displayed and a culture medium containing the VH fragment secreted by the non-suppressing host strain;
(4) immobilizing said VH fragment in the supernatant onto an appropriate support;
(5) reacting said VH fragment immobilized on the support with said VL fragment displayed on the phage in the presence or absence of an antigen;
(6) determining the amount of immobilized phages by an immunoassay using a labeled anti-phage antibody, thereby evaluating the binding ability between said VH fragment and said VL fragment; and
(7) determining that an antibody variable region in which interaction between the VH fragment and the VL fragment significantly alters in the presence of an antigen is obtained, provided that the binding ability between said VH fragment and said VL fragment in the presence of an antigen is two times or more than the binding ability between the VH fragment and the VL fragment in the absence of the antigen.

19. A method for obtaining a VL fragment of the variable region of an antibody, the method comprising the steps of:
(1) transforming a non-suppressing host strain by using the vector as described in Claim 2, or transfecting a non-suppressing host strain with a VH/VL displaying phage containing said vector;
(2) allowing the transformed non-suppressing host strain to secrete the VL fragment into the culture medium; and
(3) purifying the VL fragment from the culture medium.

20. A method for obtaining a VH fragment of the variable region of an antibody, the method comprising the steps of:
(1) transforming a non-suppressing host strain by using a vector as described in Claim 3, or transfecting a non-suppressing host strain with a VH/VL displaying phage containing said vector;
(2) allowing the transformed non-suppressing host strain to secrete the VH fragment into the culture medium; and
(3) purifying the VH fragment from the culture medium.


SEQUENCE LISTING
<110> Japan as represented by president of Tokyo university
<120> A method to determine protein interactions
<140> PCT/JP2003/010386
<141> 2003-08-15
<150> JP20020237411
<151> 2002-08-16
<160> 26
<210> 1
<211> 4301
<212> DNA
<213> Artificial Sequence
<220>
<223> Sequence of phagemid vector pKS1-HyHel10
<400>
AGCTTTGGAG CCTTTTTTTT GGAGATTTTC AACGTGAAAA AATTATTATT CGCAATTCCT 60
TTAGTTGTTC CTTTCTATTC TCACAGTGCA CAGGTCCAAC CGGCCATGGC CGAGGTGCAG 120
CTGCAGGAGT CAGGACCTAG CCTCGTGAAA CCTTCTCAGA CTCTGTCCCT CACCTGTTCT 180
GTCACTGGCG ACTCCATCAC CAGTGATTAT TGGAGCTGGA TCCGGAAATT CCCAGGGAAT 240
AGACTTGAGT ACATGGGGTA CGTAAGCTAC AGTGGTAGCA CGTACTACAA TCCATCTCTC 300
AAAAGTCGAA TCTCCATCAC TCGAGACACA TCCAAGAATC AGTACTATCT GGACCTGAAT 360
TCTGTGACTA CTGAGGACAC AGCCACATAT TACTGTGCAA ACTGGGACGG TGATTACTGG 420
GGCCAAGGGA CCACGGTCAC CGTCTCGAGC GGTGGAGGCG GTTCAATGAG TGTTTTAGTG 480
TATTCTTTCG CCTCTTTCGT TTTAGGTTGG TGCCTTCGTA GTGGCATTAC GTATTTTACC 540
CGTTTAATGG AAACTTCCTC ATGATCTAGA AGGAGATATC ATATGAAAAA GACAGCTATC 600
GCGATTGCAG TGGCACTGGC TGGTTTCGCT ACCGTTGCGC AAGCTGACTA CAAGGACGAC 660
GATGACAAAA GTGCACAGTC GACGGACATT GAGCTCACCC AGTCTCCAGC CACCCTGTCT 720
GTGACTCCAG GAAATAGCGT CAGTCTTTCC TGCAGGGCCA GCCAAAGTAT TGGCAACAAC 780
CTACACTGGT ATCAACAAAA ATCACATGAG TCTCCAAGGC TTCTCATCAA GTATGCTTCC 840
CAGTCCATCT CTGGGATCCC CTCCAGGTTC AGTGGCAGTG GATCAGGGAC AGATTTCACT 900
CTCAGTATCA ACAGTGTGGA GACTGAAGAT TTTGGAATGT ATTTCTGTCA ACAGAGTAAC 960
AGCTGGCCGT ACACGTTCGG AGGGGGGACC AAGCTGGAAA TCAAACGGGC GGCCGCACAT 1020
CATCATCACC ATCACGGGGC CGCAGAACAA AAACTCATCT CAGAAGAGGA TCTGAATGGG 1080

GCCGCATAGG TACCGCAGGT CGCGGATTTC GACACAATTT ATCAGGCGAT GATACAAATC 1140

TCCGTTGTAC TTTGTTTCGC GCTTGGTATA ATCGCTGGGG GTCAAAGATG AGAATTCACT 1200

GGCCGTCGTT TTACAACGTC GTGACTGGGA AAACCCTGGC GTTACCCAAC TTAATCGCCT 1260

TGCAGCACAT CCCCCTTTCG CCAGCTGGCG TAATAGCGAA GAGGCCCGCA CCGATCGCCC 1320

TTCCCAACAG TTGCGCAGCC TGAATGGCGA ATGGCGCCTG ATGCGGTATT TTCTCCTTAC 1380

GCATCTGTGC GGTATTTCAC ACCGCATATA AATTGTAAAC GTTAATATTT TGTTAAAATT 1440

CGCGTTAAAT TTTTGTTAAA TCAGCTCATT TTTTAACCAA TAGGCCGAAA TCGGCAAAAT 1500

CCCTTATAAA TCAAAAGAAT AGCCCGAGAT AGGGTTGAGT GTTGTTCCAG TTTGGAACAA 1560

GAGTCCACTA TTAAAGAACG TGGACTCCAA CGTCAAAGGG CGAAAAACCG TCTATCAGGG 1620

CGATGGCCCA CTACGTGAAC CATCACCCAA ATCAAGTTTT TTGGGGTCGA GGTGCCGTAA 1680

AGCACTAAAT CGGAACCCTA AAGGGAGCCC CCGATTTAGA GCTTGACGGG GAAAGCCGGC 1740

GAACGTGGCG AGAAAGGAAG GGAAGAAAGC GAAAGGAGCG GGCGCTAGGG CGCTGGCAAG 1800

TGTAGCGGTC ACGCTGCGCG TAACCACCAC ACCCGCCGCG CTTAATGCGC CGCTACAGGG 1860

CGCGTACTAT GGTTGCTTTG ACGGGTGCAC TCTCAGTACA ATCTGCTCTG ATGCCGCATA 1920

GTTAAGCCAG CCCCGACACC CGCCAACACC CGCTGACGCG CCCTGACGGG CTTGTCTGCT 1980

CCCGGCATCC GCTTACAGAC AAGCTGTGAC CGTCTCCGGG AGCTGCATGT GTCAGAGGTT 2040

TTCACCGTCA TCACCGAAAC GCGCGAGACG AAAGGGCCTC GTGATACGCC TATTTTTATA 2100

GGTTAATGTC ATGATAATAA TGGTTTCTTA GACGTCAGGT GGCACTTTTC GGGGAAATGT 2160

GCGCGGAACC CCTATTTGTT TATTTTTCTA AATACATTCA AATATGTATC CGCTCATGAG 2220

ACAATAACCC TGATAAATGC TTCAATAATA TTGAAAAAGG AAGAGTATGA GTATTCAACA 2280

TTTCCGTGTC GCCCTTATTC CCTTTTTTGC GGCATTTTGC CTTCCTGTTT TTGCTCACCC 2340

AGAAACGCTG GTGAAAGTAA AAGATGCTGA AGATCAGTTG GGTGCACGAG TGGGTTACAT 2400

CGAACTGGAT CTCAACAGCG GTAAGATCCT TGAGAGTTTT CGCCCCGAAG AACGTTTTCC 2460

AATGATGAGC ACTTTTAAAG TTCTGCTATG TGGCGCGGTA TTATCCCGTA TTGACGCCGG 2520

GCAAGAGCAA CTCGGTCGCC GCATACACTA TTCTCAGAAT GACTTGGTTG AGTACTCACC 2580

AGTCACAGAA AAGCATCTTA CGGATGGCAT GACAGTAAGA GAATTATGCA GTGCTGCCAT 2640

AACCATGAGT GATAACACTG CGGCCAACTT ACTTCTGACA ACGATCGGAG GACCGAAGGA 2700

GCTAACCGCT TTTTTGCACA ACATGGGGGA TCATGTAACT CGCCTTGATC GTTGGGAACC 2760

GGAGCTGAAT GAAGCCATAC CAAACGACGA GCGTGACACC ACGATGCCTG TAGCAATGGC 2820

AACAACGTTG CGCAAACTAT TAACTGGCGA ACTACTTACT CTAGCTTCCC GGCAACAATT 2880

AATAGACTGG ATGGAGGCGG ATAAAGTTGC AGGACCACTT CTGCGCTCGG CCCTTCCGGC 2940

TGGCTGGTTT ATTGCTGATA AATCTGGAGC CGGTGAGCGT GGGTCTCGCG GTATCATTGC 3000

AGCACTGGGG CCAGATGGTA AGCCCTCCCG TATCGTAGTT ATCTACACGA CGGGGAGTCA 3060

GGCAACTATG GATGAACGAA ATAGACAGAT CGCTGAGATA GGTGCCTCAC TGATTAAGCA 3120

TTGGTAACTG TCAGACCAAG TTTACTCATA TATACTTTAG ATTGATTTAA AACTTCATTT 3180

TTAATTTAAA AGGATCTAGG TGAAGATCCT TTTTGATAAT CTCATGACCA AAATCCCTTA 3240

ACGTGAGTTT TCGTTCCACT GAGCGTCAGA CCCCGTAGAA AAGATCAAAG GATCTTCTTG 3300

AGATCCTTTT TTTCTGCGCG TAATCTGCTG CTTGCAAACA AAAAAACCAC CGCTACCAGC 3360

GGTGGTTTGT TTGCCGGATC AAGAGCTACC AACTCTTTTT CCGAAGGTAA CTGGCTTCAG 3420

CAGAGCGCAG ATACCAAATA CTGTCCTTCT AGTGTAGCCG TAGTTAGGCC ACCACTTCAA 3480

GAACTCTGTA GCACCGCCTA CATACCTCGC TCTGCTAATC CTGTTACCAG TGGCTGCTGC 3540

CAGTGGCGAT AAGTCGTGTC TTACCGGGTT GGACTCAAGA CGATAGTTAC CGGATAAGGC 3600

GCAGCGGTCG GGCTGAACGG GGGGTTCGTG CACACAGCCC AGCTTGGAGC GAACGACCTA 3660

CACCGAACTG AGATACCTAC AGCGTGAGCA TTGAGAAAGC GCCACGCTTC CCGAAGGGAG 3720

AAAGGCGGAC AGGTATCCGG TAAGCGGCAG GGTCGGAACA GGAGAGCGCA CGAGGGAGCT 3780

TCCAGGGGGA AACGCCTGGT ATCTTTATAG TCCTGTCGGG TTTCGCCACC TCTGACTTGA 3840

GCGTCGATTT TTGTGATGCT CGTCAGGGGG GCGGAGCCTA TGGAAAAACG CCAGCAACGC 3900

GGCCTTTTTA CGGTTCCTGG CCTTTTGCTG GCCTTTTGCT CACATGTTCT TTCCTGCGTT 3960

ATCCCCTGAT TCTGTGGATA ACCGTATTAC CGCCTTTGAG TGAGCTGATA CCGCTCGCCG 4020

CAGCCGAACG ACCGAGCGCA GCGAGTCAGT GAGCGAGGAA GCGGAAGAGC GCCCAATACG 4080

CAAACCGCCT CTCCCCGCGC GTTGGCCGAT TCATTAATGC AGCTGGCACG ACAGGTTTCC 4140

CGACTGGAAA GCGGGCAGTG AGCGCAACGC AATTAATGTG AGTTAGCTCA CTCATTAGGC 4200

ACCCCAGGCT TTACACTTTA TGCTTCCGGC TCGTATGTTG TGTGGAATTG TGAGCGGATA 4260

ACAATTTCAC ACAGGAAACA GCTATGACCA TGATTACGCC A 4301

<210> 2

<211> 4294

<212> DNA

<213> Artificial Sequence

<220>

<223> Sequence of phagemid vector pKS2-HyHel10

<400>

AGCTTGCATG CAAATTCTAT TTCAAGGAGA CAGTCATAAT GAAATACCTA TTGCCTACGG 60

CAGCCGCTGG ATTGTTATTA CTCGCGGCCC AGCCGGCCAT GGCCGAGGTG CAGCTGCAGG 120

AGTCAGGACC TAGCCTCGTG AAACCTTCTC AGACTCTGTC CCTCACCTGT TCTGTCACTG i80

GCGACTCCAT CACCAGTGAT TATTGGAGCT GGATCCGGAA ATTCCCAGGG AATAGACTTG 240

AGTACATGGG GTACGTAAGC TACAGTGGTA GCACGTACTA CAATCCATCT CTCAAAAGTC 300

GAATCTCCAT CACTCGAGAC ACATCCAAGA ATCAGTACTA TCTGGACCTG AATTCTGTGA 360

CTACTGAGGA CACAGCCACA TATTACTGTG CAAACTGGGA CGGTGATTAC TGGGGCCAAG 420

GGACCACGGT CACCGTCTCG AGCGGTGGAG GCGGTTCAAT GAGTGTTTTA GTGTATTCTT 480

TCGCCTCTTT CGTTTTAGGT TGGTGCCTTC GTAGTGGCAT TACGTATTTT ACCCGTTTAA 540

TGGAAACTTC CTCATGATCT AGAAGGAGAT ATCATATGAA AAAGACAGCT ATCGCGATTG 600

CAGTGGCACT GGCTGGTTTC GCTACCGTTG CGCAAGCTGA CTACAAGGAC GACGATGACA 660

AAAGTGCACA GTCGACGGAC ATTGAGCTCA CCCAGTCTCC AGCCACCCTG TCTGTGACTC 720

CAGGAAATAG CGTCAGTCTT TCCTGCAGGG CCAGCCAAAG TATTGGCAAC AACCTACACT 780

GGTATCAACA AAAATCACAT GAGTCTCCAA GGCTTCTCAT CAAGTATGCT TCCCAGTCCA 840

TCTCTGGGAT CCCCTCCAGG TTCAGTGGCA GTGGATCAGG GACAGATTTC ACTCTCAGTA 900

TCAACAGTGT GGAGACTGAA GATTTTGGAA TGTATTTCTG TCAACAGAGT AACAGCTGGC 960

CGTACACGTT CGGAGGGGGG ACCAAGCTGG AAATCAAACG GGCGGCCGCA CATCATCATC 1020

ACCATCACGG GGCCGCAGAA CAAAAACTCA TCTCAGAAGA GGATCTGAAT GGGGCCGCAT 1080

AGGTACCGCA GGTCGCGGAT TTCGACACAA TTTATCAGGC GATGATACAA ATCTCCGTTG 1140

TACTTTGTTT CGCGCTTGGT ATAATCGCTG GGGGTCAAAG ATGAGAATTC ACTGGCCGTC 1200

GTTTTACAAC GTCGTGACTG GGAAAACCCT GGCGTTACCC AACTTAATCG CCTTGCAGCA 1260

CATCCCCCTT TCGCCAGCTG GCGTAATAGC GAAGAGGCCC GCACCGATCG CCCTTCCCAA 1320

CAGTTGCGCA GCCTGAATGG CGAATGGCGC CTGATGCGGT ATTTTCTCCT TACGCATCTG 1380

TGCGGTATTT CACACCGCAT ATAAATTGTA AACGTTAATA TTTTGTTAAA ATTCGCGTTA 1440

AATTTTTGTT AAATCAGCTC ATTTTTTAAC CAATAGGCCG AAATCGGCAA AATCCCTTAT 1500

AAATCAAAAG AATAGCCCGA GATAGGGTTG AGTGTTGTTC CAGTTTGGAA CAAGAGTCCA 1560

CTATTAAAGA ACGTGGACTC CAACGTCAAA GGGCGAAAAA CCGTCTATCA GGGCGATGGC 1620

CCACTACGTG AACCATCACC CAAATCAAGT TTTTTGGGGT CGAGGTGCCG TAAAGCACTA 1680

AATCGGAACC CTAAAGGGAG CCCCCGATTT AGAGCTTGAC GGGGAAAGCC GGCGAACGTG 1740

GCGAGAAAGG AAGGGAAGAA AGCGAAAGGA GCGGGCGCTA GGGCGCTGGC AAGTGTAGCG 1800

GTCACGCTGC GCGTAACCAC CACACCCGCC GCGCTTAATG CGCCGCTACA GGGCGCGTAC 1860

TATGGTTGCT TTGACGGGTG CACTCTCAGT ACAATCTGCT CTGATGCCGC ATAGTTAAGC 1920

CAGCCCCGAC ACCCGCCAAC ACCCGCTGAC GCGCCCTGAC GGGCTTGTCT GCTCCCGGCA 1980

TCCGCTTACA GACAAGCTGT GACCGTCTCC GGGAGCTGCA TGTGTCAGAG GTTTTCACCG 2040

TCATCACCGA AACGCGCGAG ACGAAAGGGC CTCGTGATAC GCCTATTTTT ATAGGTTAAT 2100

GTCATGATAA TAATGGTTTC TTAGACGTCA GGTGGCACTT TTCGGGGAAA TGTGCGCGGA 2160

ACCCCTATTT GTTTATTTTT CTAAATACAT TCAAATATGT ATCCGCTCAT GAGACAATAA 2220

CCCTGATAAA TGCTTCAATA ATATTGAAAA AGGAAGAGTA TGAGTATTCA ACATTTCCGT 2280

GTCGCCCTTA TTCCCTTTTT TGCGGCATTT TGCCTTCCTG TTTTTGCTCA CCCAGAAACG 2340

CTGGTGAAAG TAAAAGATGC TGAAGATCAG TTGGGTGCAC GAGTGGGTTA CATCGAACTG 2400

GATCTCAACA GCGGTAAGAT CCTTGAGAGT TTTCGCCCCG AAGAACGTTT TCCAATGATG 2460

AGCACTTTTA AAGTTCTGCT ATGTGGCGCG GTATTATCCC GTATTGACGC CGGGCAAGAG 2520

CAACTCGGTC GCCGCATACA CTATTCTCAG AATGACTTGG TTGAGTACTC ACCAGTCACA 2580

GAAAAGCATC TTACGGATGG CATGACAGTA AGAGAATTAT GCAGTGCTGC CATAACCATG 2640

AGTGATAACA CTGCGGCCAA CTTACTTCTG ACAACGATCG GAGGACCGAA GGAGCTAACC 2700

GCTTTTTTGC ACAACATGGG GGATCATGTA ACTCGCCTTG ATCGTTGGGA ACCGGAGCTG 2760

AATGAAGCCA TACCAAACGA CGAGCGTGAC ACCACGATGC CTGTAGCAAT GGCAACAACG 2820

TTGCGCAAAC TATTAACTGG CGAACTACTT ACTCTAGCTT CCCGGCAACA ATTAATAGAC 2880

TGGATGGAGG CGGATAAAGT TGCAGGACCA CTTCTGCGCT CGGCCCTTCC GGCTGGCTGG 2940

TTTATTGCTG ATAAATCTGG AGCCGGTGAG CGTGGGTCTC GCGGTATCAT TGCAGCACTG 3000

GGGCCAGATG GTAAGCCCTC CCGTATCGTA GTTATCTACA CGACGGGGAG TCAGGCAACT 3060

ATGGATGAAC GAAATAGACA GATCGCTGAG ATAGGTGCCT CACTGATTAA GCATTGGTAA 3120

CTGTCAGACC AAGTTTACTC ATATATACTT TAGATTGATT TAAAACTTCA TTTTTAATTT 3180

AAAAGGATCT AGGTGAAGAT CCTTTTTGAT AATCTCATGA CCAAAATCCC TTAACGTGAG 3240

TTTTCGTTCC ACTGAGCGTC AGACCCCGTA GAAAAGATCA AAGGATCTTC TTGAGATCCT 3300

TTTTTTCTGC GCGTAATCTG CTGCTTGCAA ACAAAAAAAC CACCGCTACC AGCGGTGGTT 3360

TGTTTGCCGG ATCAAGAGCT ACCAACTCTT TTTCCGAAGG TAACTGGCTT CAGCAGAGCG 3420

CAGATACCAA ATACTGTCCT TCTAGTGTAG CCGTAGTTAG GCCACCACTT CAAGAACTCT 3480

GTAGCACCGC CTACATACCT CGCTCTGCTA ATCCTGTTAC CAGTGGCTGC TGCCAGTGGC 3540

GATAAGTCGT GTCTTACCGG GTTGGACTCA AGACGATAGT TACCGGATAA GGCGCAGCGG 3600

TCGGGCTGAA CGGGGGGTTC GTGCACACAG CCCAGCTTGG AGCGAACGAC CTACACCGAA 3660

CTGAGATACC TACAGCGTGA GCATTGAGAA AGCGCCACGC TTCCCGAAGG GAGAAAGGCG 3720

GACAGGTATC CGGTAAGCGG CAGGGTCGGA ACAGGAGAGC GCACGAGGGA GCTTCCAGGG 3780

GGAAACGCCT GGTATCTTTA TAGTCCTGTC GGGTTTCGCC ACCTCTGACT TGAGCGTCGA 3840

TTTTTGTGAT GCTCGTCAGG GGGGCGGAGC CTATGGAAAA ACGCCAGCAA CGCGGCCTTT 3900

TTACGGTTCC TGGCCTTTTG CTGGCCTTTT GCTCACATGT TCTTTCCTGC GTTATCCCCT 3960

GATTCTGTGG ATAACCGTAT TACCGCCTTT GAGTGAGCTG ATACCGCTCG CCGCAGCCGA 4020

ACGACCGAGC GCAGCGAGTC AGTGAGCGAG GAAGCGGAAG AGCGCCCAAT ACGCAAACCG 4080

CCTCTCCCCG CGCGTTGGCC GATTCATTAA TGCAGCTGGC ACGACAGGTT TCCCGACTGG 4140

AAAGCGGGCA GTGAGCGCAA CGCAATTAAT GTGAGTTAGC TCACTCATTA GGCACCCCAG 4200

GCTTTACACT TTATGCTTCC GGCTCGTATG TTGTGTGGAA TTGTGAGCGG ATAACAATTT 4260
CACACAGGAA ACAGCTATGA CCATGATTAC GCCA 4294

<210> 3
<211> 40
<212> DNA
<213> Artificial Sequence
<220>
<223> Primer for ompA secretion signal sequence and FLAG tag
sequence (Back:ompXbaRV)
<400>
CGGGGTCGAC TGTGCACTTT TGTCATCGTC GTCCTTGTAG 40

<210> 4
<211> 40
<212> DNA
<213> Artificial Sequence
<220>
<223> Primer for ompA secretion signal sequence and FLAG tag
sequence (Forward: ompApaSalFR)
<400>
CGGGGTCGAC TGTGCACTTT TGTCATCGTC GTCCTTGTAG 40

<210> 5
<211> 29
<212> DNA
<213> Artificial Sequence
<220>
<223> Primer for KpnI site of gene VII (Back:g7KpnRV)
<400>
CGGGGGTACC GCAGGTCGCG GATTTCGAC 29

<210> 6
<211> 29
<212> DNA
<213> Artificial Sequence
<220>
<223> Primer for EcoRI site of gene VII (Forward: g7EcoFR )
<400>
CGGGGAATTC TCATCTTTGA CCCCCAGCG 29

<210> 7
<211> 50
<212> DNA
<213> Artificial Sequence
<220>
<223> Primer for XhoI site of gene IX (Back: g9XhoRV )
<400>
CGGGCTCGAG CGGTGGAGGC GGTTCAATGA GTGTTTTAGT GTATTCTTTC 50

<210> 8
<211> 33
<212> DNA
<213> Artificial Sequence
<220>
<223> Primer for XbaI site of gene IX (Forward: g9XbaFR)
<400>
CGGGTCTAGA TCATGAGGAA GTTTCCATTA AAC 33

<210> 9
<211> 42
<212> DNA
<213> Artificial Sequence
<220>
<223> Primer for split Fv linker (Back:LinkBackX)

<400>
GGGACCACGG TCACCGTCTC GAGCGGTGGA GGCGGTTCAA TG 42

<210> 10
<211> 42
<212> DNA
<213> Artificial Sequence
<220>
<223> Primer for split Fv linker (Forward: LinkFor)
<400>
AGACTGGGTG AGCTCAATGT CCGTCGACTG TGCACTTTTG TC 42

<210> 11
<211> 17
<212> DNA
<213> Artificial Sequence
<220>
<223> Primer for LacZ (Back: M13RV)
<400>
CAGGAAACAG CTATGAC 17

<210> 12
<211> 26
<212> DNA
<213> Artificial Sequence
<220>
<223> Primer for mouse VH (Forward: VH1For2X)
<400>
GACGGTGACC GTGGTCCCTT GGCCCC 26

<210> 13
<211> 24

<212> DNA
<213> Artificial Sequence
<220>
<223> Primer for mouse VL (Back: Vκ2Back )
<400>
GACATTGAGC TCACCCAGTC TCCA 24

<210> 14
<211> 17
<212> DNA
<213> Artificial Sequence
<220>
<223> Primer for M13 gene III (Forward: ReverseSEQ)
<400>
GTAAAACGAC GGCCAGT 17

<210> 15
<211> 29
<212> DNA
<213> Artificial Sequence
<220>
<223> Primer MycAKpnFor
<400>
CCGGGTACCT ATGCGGCCCC ATTCAGATC 29

<210> 16
<211> 30
<212> DNA
<213> Artificial Sequence
<220>
<223> Primer for incorporation of terminator gene (tHP1)
<400>
AGCGGTACCC GATAAAAGCG GCTTCCTGAC 30

<210> 17
<211> 30
<212> DNA
<213> Artificial Sequence
<220>
<223> Primer for incorporation of terminator gene (tHP2)
<400>
AGGAGGCCGT TTTGTTTTGC AGCCCACCTC 30

<210> 18
<211> 30
<212> DNA
<213> Artificial Sequence
<220>
<223> Primer for incorporation of terminator gene (tHP3)
<400>
GCTGAGGTGG GCTGCAAAAC AAAACGGCCT 30

<210> 19
<211> 30
<212> DNA
<213> Artificial Sequence
<220>
<223> Primer for incorporation of terminator gene (tHP4)
<400>
CCTGTCAGGA AGCCGCTTTT ATCGGGTACC 30

<210> 20
<211> 31

<212> DNA
<213> Artificial Sequence
<220>
<223> Primer for incorporation of terminator gene (tHP7)
<400>
AATTGGTACC CGATAAAAGC GGCTTCCTGA C 31

<210> 21
<211> 31
<212> DNA
<213> Artificial Sequence
<220>
<223> Primer for incorporation of terminator gene (tHP8)
<400>
AATTGAGGTG GGCTGCAAAA CAAAACGGCC T 31

<210> 22
<211> 78
<212> DNA
<213> Artificial Sequence
<220> K=G or T, M=A or C, R=A or G, Y=C or T, S=C or G, W=A
or T
<223> Forward primer for FR2 of VH (H10VHframe2)
<400>
ACCACTGTAG CTTACGTACC CCAWSYACTC CAGACSKTTA CCTGGARRTT KACGAAYCCA
GCTCCAATAA TCACTGGT 78

<210> 23
<211> 81
<212> DNA
<213> Artificial Sequence
<220> K=G or T, M=A or C, R=A or G, Y=C or T, S=C or G, W=A
or T

<223> Reverse primer for FR2 of VL (H10VLframe2)
<400>
GGCAACAACC TACACTGGTA TCAACAAAAA YMGSRCRAAT CTCCTCRGCT CCTGRTCWAK
TATGCTTCCC AGTCCATCTC T 81

<210> 24
<211> 19
<212> DNA
<213> Artificial Sequence
<220>
<223> Reverse primer for linker (H10linkRV)
<400>
GGGTACGTAA GCTACAGTG 19

<210> 25
<211> 17
<212> DNA
<213> Artificial Sequence
<220>
<223> Forward primer for linker (H10linkFR)
<400>
GATACCAGTG TAGGTTG 17

<210> 26
<211> 21
<212> DNA
<213> Artificial Sequence
<220>
<223> Reverse primer complimentary for OmpA signal sequence
(OmpARV)
<400>
ACAGCTATCG CGATTGCAGT G 21